(19) **Europäisches Patentamt**

**European Patent Office**

**Office européen des brevets**

(11) **EP 0 778 891 B1**

(12) **FASCICULE DE BREVET EUROPEEN**

(45) Date de publication et mention
de la délivrance du brevet:
**20.10.2004 Bulletin 2004/43**

(51) Int Cl.[7]: **C12N 15/13**, C07K 16/34,
A61K 39/395, C12N 15/86

(21) Numéro de dépôt: **95929923.1**

(22) Date de dépôt: **01.09.1995**

(86) Numéro de dépôt international:
**PCT/FR1995/001143**

(87) Numéro de publication internationale:
**WO 1996/007740 (14.03.1996 Gazette 1996/12)**

(54) **ANTICORPS MONOCLONAL RECOMBINANT ANTI-RHESUS D (D7C2)**

MONOKLONALER ANTIKÖRPER GEGEN RHESUS D (D7C2)

MONOCLONAL RECOMBINANT ANTI-RHESUS D (D7C2) ANTIBODY

(84) Etats contractants désignés:
**AT BE CH DE DK ES FR GB GR IE IT LI LU MC NL
PT SE**

(30) Priorité: **02.09.1994 FR 9410566**

(43) Date de publication de la demande:
**18.06.1997 Bulletin 1997/25**

(73) Titulaires:
• **INSTITUT PASTEUR
75724 Paris Cédex 15 (FR)**
• **Proteine Performance S.A.
30380 Saint-Christol-lès-Alès (FR)**

(72) Inventeurs:
• **EDELMAN, Léna
F-92100 Boulogne (FR)**
• **MARGARITTE, Christel,San Francisco General
94110 San Francisco, CA (US)**
• **KACZOREK, Michel
F-34980 Montferrier (FR)**
• **CHAABIHI, Hassan
30140 BOISSET ET GAUJAC (FR)**

(74) Mandataire: **Vialle-Presles, Marie José et al
Cabinet ORES,
36,rue de St Pétersbourg
75008 Paris (FR)**

(56) Documents cités:
**EP-A- 0 345 152          WO-A-91/07492**

• **AUSTRALIAN JOURNAL OF MEDICAL
SCIENCE;, vol. 13, no. 2, Mai 1992 pages 43-47,
STERN, D.A. ET AL.; 'Monolconal anti-RHD from
clone to consumer.'**
• **THE JOURNAL OF CLINICAL INVESTIGATION;,
vol. 90, no. 6, Décembre 1992 pages 2481-2490,
BYE, J.M. ET AL.; 'Germline variable region gene
segment derivation of human monoclonal
anti-Rh(D)'**
• **BIOFUTUR, 1993, 125, 3-15, MORELLE C
'Expression de proteines heterologues'**

EP 0 778 891 B1

**Description**

La présente Invention est relative à l'obtention en cellules d'insecte, d'un anticorps monoclonal recombinant anti-Rhésus D.

On désigne communément sous le terme "Rhésus positif", ou "Rh-positif" les sujets dont les hématies sont agglutinées par des alloanticorps dirigés contre l'antigène D (qui est l'un des antigènes du système RH), et sous le terme "Rhésus négatif", ou "Rh-négatif" les sujets dont les hématies ne sont pas agglutinées par ces alloanticorps.

La maladie hémolytique du nouveau né est due dans la majorité des cas, à la présence, chez une mère Rh-négatif, d'alloanticorps anti-RhésusD (l'allo-immunisation contre d'autres antigènes du système RH est beaucoup plus rare), qui provoquent chez un foetus Rhésus positif une anémie hémolytique qui va nécessiter, soit des transfusions *in utero*, soit une exsanguino-transfusion à la naissance dans les cas graves.

L'allo-immunisation de la mère intervient généralement lors d'un accouchement précédent ; des hématies foetales passent dans la circulation maternelle, et induisent une immunisation si l'enfant est Rh-positif.

La prévention de la maladie hémolytique du nouveau né consiste en l'injection d'anticorps anti-Rhésus D, aux femmes Rhésus négatif, immédiatement après un accouchement ou une interruption de grossesse.

Actuellement, les anticorps anti-Rhésus utilisés dans ce but sont des immunoglobulines polyclonales provenant de donneurs volontaires Rhésus négatif, immunisés à plusieurs reprises contre des hématies Rh-positif.

Ceci pose des problèmes, d'une part du fait de la nécessité de disposer de volontaires en nombre suffisant pour répondre aux besoins, et d'autre part du fait des risques de contamination par des virus ou autres pathogènes qui pourraient être présents dans les préparations d'immunoglobulines obtenues à partir du sang des volontaires.

Cependant, ce mode de production n'a à l'heure actuelle aucune alternative. En effet, bien qu'il existe des essais de culture de lignées lymphoblastoïdes, issues de lymphocytes B transformés par le virus Epstein-Barr, et secrétant des anticorps monoclonaux, aucun produit obtenu à partir de ce type de cultures n'a reçu l'autorisation de mise sur le marché du fait des risques impliqués par l'utilisation du virus Epstein-Barr.

Aussi, afin de s'affranchir des risques liés à l'utilisation du virus Epstein-Barr et d'isoler des anticorps monoclonaux anti-Rhésus D dotés de propriétés biologiques particulières, il a été mis en oeuvre une méthode d'obtention d'anticorps chimériques combinant les domaines variables et les domaines constants d'anticorps monoclonaux anti-Rhésus D dotés de propriétés différentes (WO 91/07492 publiée le 30 mai 1991 au nom de Central Blood Laboratories Authority).

Cependant, des tests effectués sur plusieurs préparations d'anticorps anti-Rhésus D disponibles dans l'art ont mis en évidence un spectre de réactivité très variable d'un anticorps à l'autre, et notamment une incapacité générale à réagir avec l'ensemble des phénotypes rhésus positif et avec le rhésus partiel Rh33 (STERN, Australian J. Medical Science 13(2) : 43-47, 1992) ; ce qui ne permet donc pas d'envisager à l'heure actuelle l'obtention d'anticorps chimériques pouvant combler l'incapacité existant au niveau du spectre de réactivité des anticorps anti-Rhésus D dont ils sont issus.

Afin d'obtenir une source d'immunoglobulines anti-Rhésus D ne présentant pas les inconvénients des immunoglobulines utilisées jusqu'à présent, les Inventeurs se sont fixé pour but la production d'un anticorps monoclonal humain anti-Rhésus recombinant dans des cellules d'insecte, en utilisant un vecteur d'expression dérivé d'un baculovirus.

Dans ce but les Inventeurs ont d'abord sélectionné une lignée produisant un anticorps monoclonal anti-Rhésus D, dénommé D7C2. Cet anticorps appartient à la classe des IgM, agglutine les hématies de phénotype Rhésus courant, les Rhésus faibles, et la plupart des Rhésus partiels à l'exception des D$^{VI}$ ; cet anticorps se lie à un polypeptide de 30 à 32 Kda de la membrane des hématies.

Les Inventeurs ont ensuite cloné les séquences codant pour les régions variables des chaînes lourde (H) et légère (L) de D7C2, et ont construit des cassettes d'expression permettant l'expression de chacune desdites séquences sous contrôle d'un promoteur fort de baculovirus.

La présente Invention a pour objet un fragment d'ADN, caractérisé en ce qu'il est choisi dans le groupe constitué par :

-    un fragment d'ADN qui comprend une séquence qui code pour la région variable de la chaîne légère de l'anticorps monoclonal D7C2 ;
-    un fragment d'ADN qui comprend une séquence qui code pour la région variable de la chaîne lourde de l'anticorps monoclonal D7C2.

Par : "séquence qui code pour la région variable de la chaîne légère de l'anticorps monoclonal D7C2", on entend en particulier la séquence identifiée dans la liste de séquences en annexe sous le numéros SEQ ID NO: 1, qui code pour le polypeptide SEQ ID N0: 2.

Par : "séquence qui code pour la région variable de la chaîne lourde de l'anticorps monoclonal D7C2", on entend en particulier la séquence SEQ ID N0: 3 qui code pour le polypeptide SEQ ID N0: 4.

Cependant, il apparaîtra à l'homme de l'art que cette définition englobe également des séquences fonctionnellement équivalentes aux séquences SEQ ID N0: 1 et SEQ ID N0: 3, à savoir, en particulier :

- toute séquence codant pour les polypeptides SEQ ID N0: 2 et SEQ ID N0: 4, et également
- des séquences codant pour des polypeptides qui peuvent différer des polypeptides SEQ ID N0: 2 et SEQ ID N0: 4 par quelques acides aminés, à condition que cette variation ne se situe pas au niveau d'une séquence peptidique impliquée dans la reconnaissance de l'épitope.

Ceci englobe par exemple une séquence codant pour un polypeptide dont la séquence diffère de celle du polypeptide SEQ ID N0: 4 par le remplacement du résidu Thr en position 23, par un résidu Ala, ce qui résulte du remplacement d'un A en position 67 dans la séquence SEQ ID NO: 3, par un G.

Des séquences fonctionnellement équivalentes aux séquences SEQ ID N0: 1 et SEQ ID N0: 3, sont également des séquences résultant d'une modification des extrémités de celles-ci en vue d'y créer des sites de restriction, ou de modifier ou supprimer ceux qui y sont présents. Ceci englobe par exemple une séquence codant pour un polypeptide dont la séquence diffère de celle du polypeptide SEQ ID N0: 2 par le remplacement des trois premiers résidus, Asp-Ile-Glu, par les résidus Gln-Ser-Val, ce qui résulte du remplacement d'un A en position 67 dans la séquence SEQ ID NO: 3, par un G.

La présente Invention a également pour objet une cassette d'expression comprenant une séquence codant pour la région variable de la chaîne légère de l'anticorps monoclonal D7C2, ou une séquence codant pour la région variable de la chaîne lourde de l'anticorps monoclonal D7C2, laquelle séquence est placée sous contrôle transcriptionnel d'un promoteur approprié.

Selon un mode de réalisation préféré de la présente Invention, ledit promoteur est un promoteur de baculovirus.

A titre d'exemple de promoteurs de baculovirus utilisables pour la mise en oeuvre de la présente Invention, on citera les promoteurs de la polyédrine et de P10 des baculovirus AcMNPV ou SIMNPV, ou des dérivés de promoteurs de baculovirus, constitués par des promoteurs synthétiques ou recombinants, obtenus à partir d'un promoteur de baculovirus, et fonctionnels en cellules d'insectes, tel que par exemple celui décrit par WANG et al, [Gene, 100, 131-137, (1991)].

Une cassette d'expression conforme à ce mode de réalisation comprend par exemple:

- un promoteur de baculovirus, tel que défini ci-dessus ;
- une séquence codant pour un peptide signal de sécrétion ;
- une séquence codant pour le domaine variable de la chaîne légère de l'anticorps monoclonal D7C2 et une séquence codant pour le domaine constant de la chaîne légère d'une immunoglobuline ; ou
- une séquence codant pour le domaine variable de la chaîne lourde de l'anticorps monoclonal D7C2 et une séquence codant pour le domaine constant de la chaîne lourde d'une immunoglobuline.

Un grand nombre de séquences codant pour des peptides signal fonctionnels en cellules d'insecte sont utilisables pour la mise en oeuvre de la présente Invention. A titre d'exemple non limitatif, on citera les séquences codant pour les peptides signal de l'acétylcholinestérase de Drosophile, de la trophoblastine ovine, ainsi que les séquences codant pour les peptides signal des chaînes H et L d'immunoglobulines murines ou humaines, etc...

Chacune des cassettes conformes à l'invention permet l'expression, soit de la chaîne légère, soit de la chaîne lourde, d'un anticorps recombinant, dénommé ci-après r-D7C2, possédant la spécificité de l'anticorps monoclonal D7C2.

Selon une disposition préférée de ce mode de réalisation, les séquences codant pour les domaines constants des chaînes légères et lourdes d'immunoglobuline sont des séquences d'origine humaine.

On peut choisir la séquence codant pour le domaine constant de la chaîne légère parmi les séquences codant pour les domaines constants des chaînes légères kappa ($\kappa$) et lambda ($\lambda$).

On peut choisir la séquence codant pour le domaine constant de la chaîne lourde parmi les séquences codant pour les domaines constants des chaînes lourdes $\gamma 1$, $\gamma 2$, $\gamma 3$, $\gamma 4$, $\alpha$, $\epsilon$, et $\mu$. On peut ainsi obtenir un anticorps recombinant appartenant à la classe d'immunoglobuline (IgG1, IgG2, IgG3, IgG4, IgA, IgE, IgM) que l'on souhaite.

Très avantageusement, on choisira une séquence codant pour le domaine constant C$\gamma$ d'une chaîne lourde $\gamma$. L'anticorps recombinant r-D7C2 obtenu de la sorte, appartient à la classe des IgG, et sera dénommé ci-après r-IgG-D7C2.

Les séquences codant pour la chaine légère et pour la chaîne lourde d'un anticorps r-IgG-D7C2, d'isotype IgG1, sont respectivement représentées dans la liste de séquence en annexe sous les numéros SEQ ID N0: 5 et SEQ ID N0: 7 ; les séquences polypeptidiques correspondantes portent les numéros d'identification respectifs SEQ ID N0:

6 et SEQ ID N0: 8.

La présente Invention a également pour objet des vecteurs recombinants, portant au moins une cassette d'expression telle que définie ci-dessus ; dans ce cadre, la présente Invention englobe en particulier des baculovirus recombinants permettant l'expression de l'anticorps r-D7C2, ainsi que des plasmides de transfert permettant la construction desdits baculovirus recombinants.

Les plasmides de transfert conformes à l'invention portent un insert comprenant : une cassette d'expression telle que définie ci-dessus, et de part et d'autre de cette cassette, des séquences de baculovirus homologues de celles des régions flanquant la portion du génome viral en remplacement de laquelle on souhaite insérer ladite cassette.

Selon un mode de réalisation préféré des plasmides de transfert conformes à la présente Invention, lesdites séquences de baculovirus sont homologues de celles des régions flanquant le gène de la p10, ou homologues de celles des régions flanquant le gène de la polyédrine.

Selon une disposition particulièrement avantageuse de ce mode de réalisation, la cassette d'expression contenant le gène codant pour la chaîne légère de l'anticorps r-D7C2 est flanquée des régions entourant le gène de la polyédrine dans le baculovirus sauvage, et la cassette d'expression portant le gène codant pour la chaîne lourde de l'anticorps r-D7C2 est flanquée des régions entourant le gène P10 dans le baculovirus sauvage.

Schématiquement, la construction des plasmides de transfert conformes à l'invention se fait en insérant dans un plasmide capable de se répliquer chez un hôte bactérien (en général *E. coli*), la région du gène de baculovirus (par exemple p10 ou polyédrine, ou tout autre locus du baculovirus) à la place duquel on souhaite insérer les gènes codant pour les chaînes H ou L d'immunoglobuline. Dans cette région, la séquence codante du gène de baculovirus (et éventuellement la séquence promoteur dudit gène) est remplacée par la séquence codant pour la chaîne d'immunoglobuline à exprimer (et éventuellement par la séquence du promoteur sous contrôle duquel on souhaite exprimer cette chaîne d'immunoglobuline, s'il s'agit par exemple d'un promoteur "dérivé"). Le plasmide de transfert ainsi obtenu contient donc un insert comprenant une séquence hétérologue (séquence d'une chaîne de l'anticorps r-D7C2) flanquée de séquences de baculovirus.

Pour permettre l'expression simultanée de la chaîne lourde (chaîne H) et de la chaîne légère (chaîne L) et leur réassociation pour former la molécule d'anticorps recombinant, les Inventeurs ont inséré les deux cassettes sur un même vecteur d'expression. Ils ont ainsi obtenu un baculovirus double recombinant dans lequel la séquence codante de chacune des chaînes H et L est sous le contrôle d'un promoteur fort.

Un baculovirus recombinant conforme à l'Invention, permettant l'expression de l'anticorps r-D7C2, peut être construit selon le principe suivant :

- l'on prépare séparément deux plasmides de transfert, tels que définis ci-dessus : un pour la chaîne H, et un pour la chaîne L.

On cotransfecte ensuite les cellules d'insecte avec l'ADN des vecteurs de transfert ainsi réalisés et l'ADN du baculovirus. Cette cotransfection est effectuée en deux étapes :

Le plasmide de transfert contenant la cassette d'expression pour le gène de la chaîne légère flanquée des régions entourant le gène de la polyédrine dans le baculovirus sauvage, est utilisé, avec l'ADN de baculovirus sauvage AcMNPV, pour cotransfecter des cellules d'insecte en culture.

Par recombinaison homologue entre l'ADN viral et le plasmide, les séquences codantes de la chaîne légère de l'immunoglobuline recombinante sont transférées dans le génome viral.

Après réplication de l'ADN viral dans les cellules transfectées, l'on procède à la sélection des baculovirus recombinants ayant intégré la séquence de la chaîne légère de l'immunoglobuline recombinante.

Ces baculovirus recombinants sont sélectionnés selon deux critères : leur incapacité à produire des polyèdres et leur capacité à exprimer la chaîne légère.

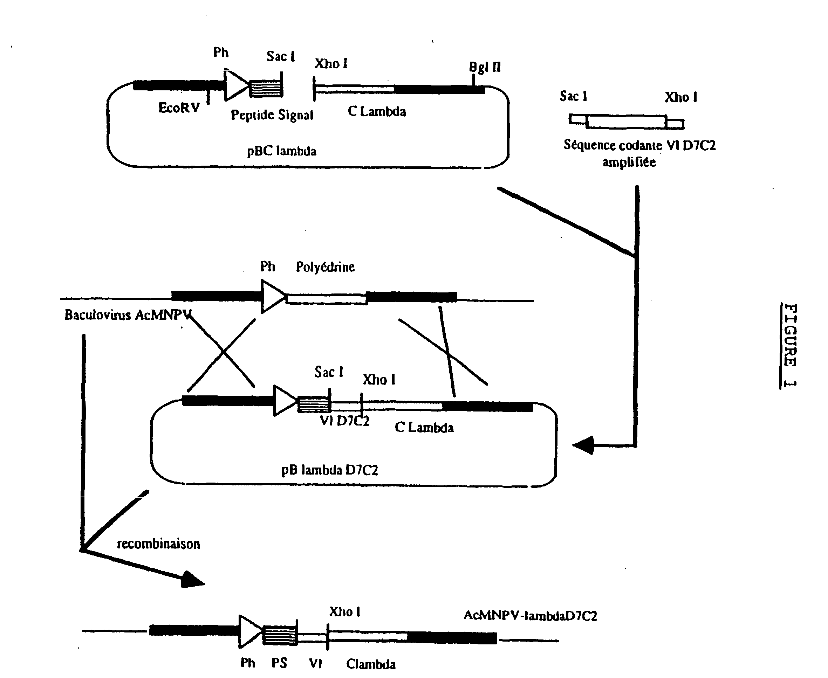

La Figure 1 illustre l'obtention du baculovirus exprimant la chaîne légère lambda de l'anticorps recombinant r-IgG-D7C2.

Dans une deuxième étape, les cellules sont cotransfectées avec l'ADN du baculovirus recombinant obtenu à l'issue de la première étape, et avec celui du plasmide de transfert contenant la cassette d'expression portant le gène codant pour la chaîne lourde de l'anticorps recombinant r-D7C2 flanquée des régions entourant le gène P10 du baculovirus. Par recombinaison homologue, comme précédemment, le gène de la chaîne lourde est transféré dans l'ADN viral.

L'on sélectionne alors les virus double-recombinants qui sont capables de produire simultanément une chaîne lourde et une chaîne légère d'immunoglobuline. La détection de la chaîne lourde et de la chaîne légère d'immunoglobuline est effectuée par ELISA.

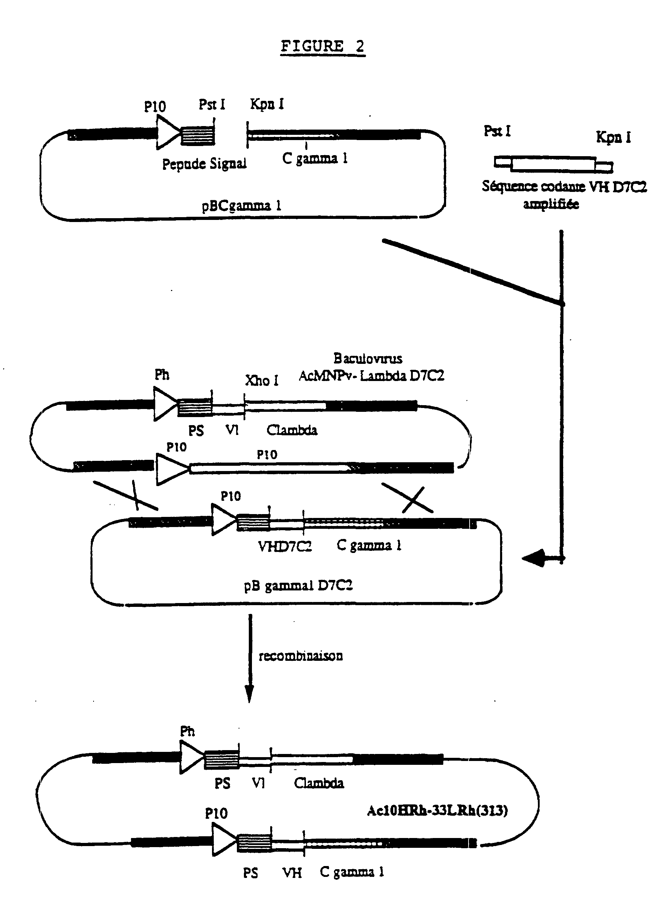

La Figure 2 illustre cette deuxième étape qui permet d'obtenir un baculovirus exprimant la chaîne légère lambda et la chaîne lourde gamma de l'anticorps recombinant r-IgG-D7C2.

La présente Invention a pour objet un baculovirus recombinant caractérisé en ce qu'il comprend au moins une cassette d'expression, telle que définie ci-dessus, comprenant une séquence codant tout ou partie de la chaîne H ou une séquence codant pour tout ou partie de la chaîne L de l'anticorps r-D7C2, laquelle séquence est placée sous contrôle transcriptionnel d'un promoteur fort de baculovirus.

Selon un mode de réalisation d'un baculovirus recombinant conforme à l'invention il comprend une cassette d'expression comprenant la séquence codant pour la chaîne H et une cassette d'expression comprenant la séquence codant pour la chaîne L, de l'anticorps monoclonal r-D7C2.

Selon une disposition préférée de ce mode de réalisation, le promoteur contrôlant la transcription de la séquence codant pour la chaîne L de l'anticorps monoclonal r-D7C2, et le promoteur contrôlant la transcription de la séquence codant pour la chaîne H de l'anticorps monoclonal r-D7C2, sont deux promoteurs différents.

Selon une modalité avantageuse de cette disposition, l'un dedits promoteurs est situé à l'emplacement occupé chez le baculovirus sauvage, par le promoteur de la polyédrine et l'autre est situé à l'emplacement occupé chez le baculovirus sauvage, par le promoteur de la P10.

Selon encore une autre modalité avantageuse de cette disposition, l'un des promoteurs est le promoteur de la polyédrine ou l'un de ses dérivés, et l'autre est le promoteur de la p10 ou l'un de ses dérivés.

Avantageusement, le promoteur contrôlant la transcription de la séquence codant pour la chaîne légère de l'anticorps r-D7C2 est le promoteur de la polyédrine ou l'un de ses dérivés, et le promoteur contrôlant la transcription de la séquence codant pour la chaîne lourde de l'anticorps r-D7C2 est le promoteur de la P10 ou l'un de ses dérivés.

Selon une autre disposition de ce mode de réalisation, la séquence codant pour le peptide signal associé à la chaîne L de l'anticorps monoclonal r-D7C2, et la séquence codant pour le peptide signal associé à la chaîne H de l'anticorps monoclonal r-D7C2, sont deux séquences différentes.

Un baculovirus recombinant conforme à l'Invention, qui comprend une cassette d'expression comprenant la séquence codant pour la chaîne λ et une cassette d'expression comprenant la séquence codant pour la chaîne γ1 de l'anticorps monoclonal r-IgG-D7C2, a été déposé le 19 août 1994, auprès de la C.N.C.M. (Collection Nationale de Cultures de Microorganismes, tenue par l'Institut Pasteur, 25 rue du Docteur ROUX, Paris), sous le numéro I-1468.

La présente Invention englobe également des cellules d'insecte infectées avec un baculovirus recombinant conforme à l'invention.

L'expression et la production de l'anticorps monoclonal recombinant r-D7C2 est obtenue *in vitro* dans des cellules d'insecte conformes à l'invention.

L'infection des cellules par un baculovirus double recombinant conforme à l'Invention résulte dans la production simultanée des chaînes H et L. Ces chaînes s'assemblent pour reconstituer l'anticorps monoclonal recombinant r-D7C2 qui est par la suite sécrété dans le milieu de culture.

La présente Invention a également pour objet un procédé de préparation d'un anticorps monoclonal recombinant, caractérisé en ce qu'il comprend une étape au cours de laquelle l'on cultive des cellules d'insecte infectées avec un baculovirus recombinant conforme à l'invention, et une étape au cours de laquelle l'on obtient ledit anticorps à partir du milieu de culture.

La présente Invention a également pour objet un anticorps monoclonal recombinant, caractérisé en ce que ses domaines variables ont la séquence des domaines variables de l'anticorps monoclonal D7C2 ; ceci englobe en particulier les préparations d'anticorps monoclonal recombinant r-D7C2 susceptibles d'être obtenues par le procédé conforme à l'invention, défini ci-dessus.

Par : "séquence des domaines variables de l'anticorps monoclonal D7C2", on entend en particulier la séquence du domaine variable de la chaîne légère de l'anticorps monoclonal D7C2 identifiée dans la liste de séquences en annexe sous le numéro SEQ ID N0: 2, et la séquence du domaine variable de la chaîne lourde de l'anticorps monoclonal D7C2 identifiée dans la liste de séquences en annexe sous le numéro SEQ ID N0: 4.

Selon un mode de réalisation préféré de l'anticorps monoclonal recombinant r-D7C2, il appartient à la classe des IgG.

Selon une disposition préférée de ce mode de réalisation, ledit anticorps r-D7C2 est d'isotype IgG1.

Un anticorps recombinant conforme à l'Invention peut être utilisé pour le diagnostic, ou pour la thérapeutique. Une utilisation particulièrement intéressante concerne l'obtention de médicaments destinés à la prévention de la maladie hémolytique du nouveau-né.

Dans ce but, on utilisera en particulier, un anticorps recombinant r-IgG-D7C2, de classe IgG, qui possède une activité effectrice ADCC, dont est dépourvu l'anticorps parental D7C2, qui est une IgM.

La production d'un anticorps monoclonal anti-Rhésus D en cellules d'insecte permet d'éviter les risques et les problèmes liés à l'immunisation de volontaires masculins Rh-D négatifs par injection de globules rouges humains RhD positifs, et de diminuer les risques de contamination par des virus ou autre pathogènes qui pourraient être présents dans les préparations d'immunoglobulines extraites du sang des volontaires.

En outre, ce mode de production présente les avantages suivants :

- sécurité pharmaceutique : l'utilisation de lignées cellulaires d'insecte et de baculovirus ne comporte pas le risque de virus adventices ou d'oncogènes qui se présente lorsque l'on utilise des lignées humaines ;

- standardisation du procédé de production, et du produit qui en est issu ;
- source d'approvisionnement en quantité quasi-illimitée.

La présente Invention sera mieux comprise à l'aide du complément de description qui va suivre, qui se réfère à des exemples de préparation de baculovirus recombinants conformes à l'invention, et à leur utilisation pour la production d'un anticorps recombinant d'isotype IgG1, dénommé ci-après r-IgG-D7C2, dans des cellules d'insectes.
Il doit être bien entendu toutefois que ces exemples sont donnés uniquement à titre d'illustration de l'objet de l'Invention dont ils ne constituent en aucune manière une limitation.

**Exemple 1. : Obtention de l'anticorps D7C2, et clonage des chaînes lourdes et légères.**

A) Description de l'anticorps D7C2 :

La lignée humaine D7C2 a été obtenue après immortalisation par le virus Epstein-Barr de lymphocytes de sang périphérique provenant d'un donneur Rhésus négatif (dce/dce) immunisé à 5 reprises avec des hématies Rhésus positif (DCe/DCe).
L'anticorps secrété par cette lignée est d'isotype IgM. Cet anticorps qui sera dénommé ci-après IgM-D7C2, agglutine les hématies de phénotype Rhésus positif DCcee; DCCee, DccEE, Dccee, et n'agglutine pas les hématies Rhésus négatif ddccee, ddCcee, ddccEe. Il agglutine les rhésus faibles, et la plupart des rhésus partiels (agglutination de DIIIa, DIIIc, DIVA, DVa, DVc, DVII, et Rh33), à l'exception des DVI. Il agglutine les hématies LW(a-b+), et n'agglutine pas les hématies r$^G$ et Rhmod. Les tests d'immuno-précipitation effectués sur des préparations de membranes de globules rouges Rhésus positif DCe/DCe solubilisées au TRITON X-1OO ont montré une liaison de l'anticorps IgM-D7C2 avec un polypeptide de 30 à 32 Kda environ.

B) Clonage et séquençage des parties variables de la chaîne lourde et de la chaîne légère

L'ARN total est extrait des cellules du clone D7C2 par le thiocyanate de guanidine, puis précipité à l'isopropanol. Après élimination de l'ADN par digestion à la DNAse, l'ARN est précipité par NaCl (concentration finale 0,2M)
L'ADNc est synthétisé à partir de 1μg d'ARN par transcription inverse, en utilisant une amorce oligo-dT.
les parties variables des chaînes lourdes (VH) et légères (VL) sont amplifiées par ACP (amplification en chaîne par polymérase) à partir de 1μg d'ADNc simple brin, en utilisant la Taq DNA polymérase.
Les amorces utilisées sont les suivantes :

\*    **Pour VH :.**

```
VH1 (SEQ ID NO : 9) :
CCTCAGTGAAGGTCTCCTGCAAGG ;


VH2 (SEQ ID NO : 10) :
TCCTGCGCTGGTGAAAGCCACACA ;


VH3 (SEQ ID NO : 11) :
GGTCCCTGAGACTCTCCTGTGCA ;


VH4a (SEQ ID NO : 12) :
TCGGAGACCCTGTCCCTCACCTGCA ;
```

```
VH4b (SEQ ID N0 : 13) :
CGCTGTCTCTGGTTACTCCATCAG ;


VH5 (SEQ ID N0 : 14) :
GAAAAAGCCCGGGGAGTCTCTGAA ;


VH6 (SEQ ID N0 : 15) :
CCTGTGCCATCTCCGGGGACAGTG ;
```

\*    **Pour VL :**

```
              VλFW1 (SEQ ID N0 : 16) :
              CAGTCTGTGCTGACTCAG ;


              Cλ (SEQ ID N0 : 17) :
              CACACYAGTGTRGCCTGGTT.
```

Trente cycles d'amplification sont ainsi effectués.

Après phosphorylation, le fragment amplifié est purifié sur gel d'agarose, puis ligaturé dans le plasmide PTZ18R. Le plasmide obtenu est utilisé pour transformer *Escherichia coli* KL1 blue.

La sélection des bactéries transformées est faite par un test de résistance à l'ampicilline.

La séquence nucléotidique des inserts des vecteurs plasmidiques respectivement dénommés PTZ-$V_L$ D7C2 et PTZ-$V_H$D7C2 est déterminée à l'aide du kit de séquencage PROMEGA.

La séquence codant pour la région variable de la chaîne légère de l'anticorps monoclonal humain IgM-D7C2 est représentée dans la liste de séquence en annexe sous le numéro SEQ ID N0 : 1 ; la séquence polypeptidique correspondante est représentée dans la liste de séquence en annexe sous le numéro SEQ ID N0 : 2.

La séquence codant pour la région variable de la chaîne lourde de l'anticorps monoclonal humain IgM-D7C2 est représentée dans la liste de séquences en annexe sous le numéro SEQ ID N0 : 3 ; la séquence polypeptidique correspondante est représentée dans la liste de séquence en annexe sous le numéro SEQ ID N0 : 4.

Les emplacements des CDR (régions déteminant la complémentarité) sont indiqués dans les caractéristiques des séquences SEQ ID N0 : 1 et SEQ ID N0 : 3.

**Exemple 2 : Construction de plasmides de transfert portant les séquences variables de D7C2**

A) PLASMIDE DE TRANSFERT POUR LA CHAINE LEGERE LAMBDA :

1) Obtention du plasmide pBCλ

a- Plasmide pGmAc116T :

Ce vecteur est dérivé du plasmide pGmAc115T [ROYER et al., J. Virol., 66, 3230-3235, (1992)], lui-même dérivé du plasmide pAcl [CHAABIHI et al., J. Virol., 67, 2664-2671 (1993)] contenant le fragment EcoRI-I du baculovirus de la polyédrose nucléaire d'*Autographa californica* (AcMNPV), et donc le gène polyédrine, et les séquences flanquant ledit gène. Pour obtenir pGmAc116T, le plasmide pGmAc115T a été délété d'un fragment de 1900pb allant d'un site EcoRI situé en amont du gène polyédrine à un site XhoI situé 1900pb en aval de ce site EcoRI.

5 μg du plasmide pGmAc115T ont été digérés pendant 2 heures à 37°C par 15 unités d'enzyme XhoI (BOEHRINGER), dans un volume réactionnel de 50 μl et dans les conditions préconisées par le fournisseur. Après extraction au phénol/chloroforme, l'ADN plasmidique a été précipité à l'alcool. Cet ADN a été ensuite partiellement coupé par EcoRI (BOEHRINGER) dans un volume réactionnel de 50 µl en présence de 0,5 unité d'enzyme. L'incubation a été faite à 37°C pendant 20 minutes. Après une nouvelle extraction au phénol/chloroforme, les extrémités générées par les coupures XhoI et EcoRI ont été rendues franches par l'enzyme de Klenow (BIOLABS) en présence des 4 dNTPs selon le protocole préconisé par le fournisseur. L'ADN plasmidique a enfin été précipité à l'alcool et incubé avec la ligase du phage T4 (BOEHRINGER) dans les conditions préconisées par le fournisseur.

Des bactéries *E. coli* compétentes ont été transformées par une partie du mélange de ligation ; le criblage des colonies issues de cette transformation a permis de sélectionner le plasmide pGmAc116T. Ce plasmide contient un site BglII en aval du promoteur de la polyédrine.

b- Peptide signal :

La séquence codante choisie pour le peptide signal a été synthétisée chimiquement sous forme de deux oligonucléotides complémentaires ayant des extrémités permettant l'insertion du duplex dans un site BglII. Pour l'appariement, 15 µg de chacun des deux oligonucléotides sont incubés dans 50 µl de tampon (Tris 1 mM pH 7,5, EDTA O,1 mM), pendant 5 minutes dans un bain marie à 70°C. Le bain est ensuite laissé refroidir jusqu'à température ambiante (22 à 25°C). Le produit d'appariement est utilisé directement dans les réactions de ligation avec le plasmide pGmAc116T préalablement coupé par BglII.

Les conditions de ligation sont les suivantes :

1µg du plasmide pGmAc116T coupé par BglII ; 1µg de l'oligonucléotide bicaténaire portant la séquence codant pour le peptide signal ; 2µl de tampon ligase 10X (BOEHRINGER) ; eau distillée q.s.p. 19µl ; 1 unité (1µl) de ligase (BOEHRINGER). L'incubation est effectuée à 22°C pendant 2 heures ; le produit de ligation est utilisé pour transformer des bactéries *E.coli* compétentes.

c- Région constante Cλ :

La séquence codante de la région constante de la chaîne légère λ humaine a été amplifiée par ACP en utilisant comme matrice de l'ADNc de lymphocytes B humains. Les lymphocytes humains (environ 5x10[8]) ont été préparés à partir de 200 ml de sang en utilisant HISTOPAQUE® (SIGMA). L'ARN total a été extrait de ces lymphocytes en utilisant un kit PHARMACIA (RNA extraction kit). Le premier brin d'ADNc a été préparé à partir de l'ARN total à l'aide du kit "First-Strand cDNA synthesis kit" de PHARMACIA.

L'amplification par ACP de la région Cλ a été réalisée en présence de l'amorce OPP-HuCλ3' complémentaire de l'extrémité 3' des régions Cλ et apportant le site de restriction BglII et de l'amorce OPP-HuCλ5' complémentaire de l'extrémité 5' des régions Cλ et apportant le site de restriction XhoI.

Les séquences des deux amorces sont les suivantes :

```
*OPP-HuCλ3' (SEQ ID NO : 18) :
5'-CCT GTC AGA TCT ATG AAC ATT CTG TAG GGG-3'
(site BglII souligné)


*OPP-HuCλ5' (SEQ ID NO : 19) :
5'-CCG CCC TCC CTC GAG CTT CAA-3'
(site XhoI souligné)
```

Le produit de l'amplification a été digéré par BglII et XhoI avant d'être cloné dans les sites XhoI-BglII du plasmide pGmAc portant la séquence codant pour le peptide signal.

La composition du mélange de ligation est la suivante :

1µg du plasmide pGmAc coupé par XhoI et BglII ; 200 ng du fragment Cλ amplifié et digéré par BglII et XhoI, 2µl de tampon ligase 10X (BOEHRINGER), eau distillée q.s.p. 19µl, 1 unité (1µl) de ligase (BOEHRINGER).

L'incubation est effectuée à 22°C pendant 2 heures ; le produit de ligation est utilisé pour transformer des bactéries *E.coli* compétentes.

Le plasmide obtenu est dénommé pBCλ.

2) Clonage de la région variable de la chaîne λ de D7C2

Le clonage de la région variable de la chaîne légère λ de l'anticorps monoclonal humain IgM-D7C2 a été réalisé de la manière suivante :

La région variable λ de l'anticorps monoclonal IgM-D7C2 a été amplifiée par ACP en utilisant :

- comme matrice, l'ADN du plasmide PTZ-V$_L$D7C2 décrit à l'exemple 1 ci-dessus, qui porte la région variable de la chaîne légère de l'anticorps monoclonal IgM-D7C2 ;
- une amorce représentant une séquence consensus à l'extrémité 5' des gènes V$_L$ humains (OPP-HuVλ5'), et une autre amorce complémentaire de la région 5' de la séquence codant pour la région constante lambda (OPP-HuVλ3'), et qui permet d'amplifier toute région variable de chaîne λ).

Ces amorces apportent en outre respectivement les sites de restriction enzymatiques SacI et XhoI.

Les séquences nucléotidiques de ces amorces sont les suivantes :

```
* OPP-HuVλ5' (SEQ ID N0 : 20) :
5' -CA(GC)TCTGAGCTCAC(GT)CAG- 3'
(site SacI souligné)
```

L'utilisation de cette amorce provoque la modification de la séquence Gln-Ser-Val des trois premiers aminoacides de la charpente 1 de l'anticorps parental IgM-D7C2 en Asp-Ile-Glu dans l'anticorps recombinant.

```
* OPP-HuVλ3' (SEQ ID N0 : 21) :
5' -TTGAAGCTCGAGGGAGGGCGGGAA- 3'
(site XhoI souligné)
```

La composition du mélange d'amplification est la suivante :

10 μl de tampon 10X de l'ADN polymérase ; 100 ng de plasmide PPTZ-V$_L$D7C2 ; 4 μl du mélange de déoxynucléotides (mélange contenant 5 mM dATP + 5 mM dCTP + 5 mM dGTP + 5 mM dTTP, BOEHRINGER) ; 2 mM MgSO4 ; 1000 pmoles de l'amorce OPP-HuVλ5' et 200 pmoles de l'amorce OPP-HuVλ3'; 1 μl d'ADN polymérase thermostable, et eau distillée q.s.p. 100 μl.

L'amplification a été réalisée en 30 cycles successifs d'incubation à 95°C pendant 30 secondes, 40°C pendant 30 secondes, et 72°C pendant 30 secondes, puis a été suivie d'une incubation à 72°C pendant 10 minutes.

L'amplification a permis d'obtenir un fragment d'environ 360 pb contenant la région variable V$_L$ de l'anticorps D7C2 ainsi que la séquence codant pour les 16 premiers acides aminés de la région constante humaine λ.

Après amplification de la région V$_L$, une digestion par les enzymes SacI et XhoI du produit d'amplification a été réalisée et le fragment obtenu a été inséré entre les sites SacI et XhoI du plasmide pBCλ pour donner le plasmide pBλD7C2. La composition du mélange de ligation est la suivante :

2 μl de tampon 10X pour la T4-DNA ligase (BOEHRINGER) ; 100 ng du fragment V$_L$D7C2 traité par les enzymes SacI et XhoI, 1 μl du plasmide pBCλ coupé par SacI et XhoI ; 1 μl de ligase (1 unité), et eau distillée q.s.p. 20 μl.

L'incubation est effectuée à 16°C pendant 8 heures puis le produit de ligation est utilisé pour transformer des bactéries *E. coli* compétentes.

Les étapes de construction du plasmide pBλD7C2 sont représentées à la Figure 1.

La séquence de l'insert du plasmide pBλD7C2 qui code pour la chaine légère lambda d'un anticorps r-D7C2, et pour le peptide signal, est représentée dans la liste de séquence en annexe sous le numéro SEQ ID N0: 5 ; la séquence polypeptidique de la chaîne légère lambda codée par cet insert est représentée sous le numéro SEQ ID N0: 6.

B) PLASMIDE DE TRANSFERT POUR LA CHAINE LOURDE

1) Obtention du plasmide pBCγ1

a- Plasmide de transfert :

Le plasmide pGm16 [BLANC et al, Virology, 192, 651-654, (1993)] dérive d'un plasmide dans lequel a été cloné le fragment EcoRI-P du baculovirus AcMNPV contenant le gène p10. La quasi-totalité de la séquence codante a été délétée et remplacée par un site BglII permettant l'insertion de séquences à exprimer sous le contrôle du promoteur p10.

b- Peptide signal :

La séquence codante de ce peptide est celle d'un gène VH de souris (NEUBERGER M.S., 1983. EMBO J, 2, 1373-1378).
Elle a été synthétisée chimiquement sous forme de brins complémentaires, de manière à ce qu'elle puisse être insérée dans un site BglII (Figure 1). Les conditions d'appariement et de ligation sont identiques à celles utilisées pour la chaîne légère.

c- Régions constantes humaines (Cγ1):

Le cDNA de la séquence codante de la région Cγ1 humaine a été amplifié par ACP en utilisant les amorces suivantes :

```
*HuCγlBAC (SEQ ID N0 : 22) :
5' CAA GGT ACC ACG GTC ACC GTC TCC - 3'
(site KpnI souligné).
```

Cette amorce correspond à une séquence consensus des régions JH murines (extrémités 3' des régions variables des chaînes lourdes murines), et comprend un site KpnI.

```
*HuCγ1FOR (SEQ ID N0 : 23) :
5'-GAAGATC TCA TTT ACC CGG AGA CAG GGA G-3'
(site BglII souligné).
```

La séquence a été déterminée à partir de séquences Cγ1 humaines. L'amorce est complémentaire de l'extrémité 3' des Cγ1 humaines, et permet de reconstituer après amplification un site BglIII en aval du codon stop.
La matrice utilisée pour amplifier la région Cγ1 humaine est le même mélange d'ADNc que celui utilisé pour l'amplification des séquences codantes Cλ
Le produit d'amplification a été séquencé et cloné dans le vecteur de transfert pGm16 portant la séquence codant pour le peptide signal. La construction obtenue a été appelée pBCγ1.

2) Clonage de la région variable de la chaîne lourde de l'anticorps D7C2

Le clonage de la région variable $V_H$ de la chaîne lourde de l'anticorps D7C2 a été réalisé de la manière suivante :
La région variable $V_H$ de la chaîne lourde de l'anticorps monoclonal IgM-D7C2 a été amplifiée par ACP en utilisant :

- comme matrice l'ADN du plasmide PTZ-$V_H$D7C2 portant la région variable de la chaîne lourde de l'anticorps monoclonal IgM-D7C2,
- une amorce reconstituant l'extrémité 5' des régions variables de la famille VH4 (OC15-HuVH4) et une seconde amorce complémentaire de la partie 3' des gènes JH humains (OPP-HuJH). Ces amorces apportent respectivement les sites de restriction enzymatiques PstI et KpnI.

Les séquences nucléotidiques de ces amorces sont les suivantes :

```
* OC15-HuVH4 (SEQ ID NO : 24) :
5' - GTC CAA CTG CAG CAG TGG GGC GCA GGA CTG
TTG AAG CCT TCG GAG ACC CTG TCC CTC - 3'
(le site PstI est souligné)
```

Cette amorce a été déterminée pour reconstituer la séquence codant pour les 14 premiers acides aminés de la charpente 1 des régions variables de la famille VH4 des chaînes λ1 humaines, qui manquait dans le plasmide PTZ-V$_H$D7C2.

```
* OPP-HuJH (SEQ ID NO : 25) :
5'- TGA GGA GAC GGT GAC CGT GGT ACC TTG GC- 3'
(Le site KpnI est souligné).
```

Cette amorce est complémentaire de la séquence consensus à l'extrémité 3' des régions JH humaines et comprend un site KpnI

La composition du mélange d'amplification est la suivante :

10 μl de tampon 10X de l'ADN polymérase, 100 ng de plasmide PTZ-V$_H$D7C2, 4 μl du mélange de déoxynucléotides (mélange contenant 5 mM dATP + 5 mM dCTP + 5 mM dGTP + 5 mM dTTP, BOEHRINGER), 75 pmoles de l'amorce OC15-HuVH4 et 100 pmoles de l'amorce OPP-HuJH, 1μl de DNA polymérase thermostable et eau distillée q.s.p. 100 μl.

L'amplification a été effectuée en 30 cycles successifs d'incubation à 95°C pendant 30 secondes, 55°C pendant 30 secondes et 72°C pendant 30 secondes et a été suivie d'une incubation à 72°C pendant 10 minutes.

Après amplification de la région V$_H$D7C2, une digestion Pst I-KpnI du fragment d'amplification obtenu (taille environ 360 pb) a été réalisée et le fragment obtenu a été inséré entre les sites PstI et KpnI du plasmide cassette chaîne lourde pBCγ1 pour donner le plasmide chargé pBγ1D7C2.

La composition du mélange de ligation est la suivante :

2 μl de tampon 10X de la ligase (BOEHRINGER), 20 ng du fragment V1D7C2 traité par les enzymes PstI et KpnI, 500 ng du plasmide pBCλ1 coupé par PstI et KpnI, 1μl de ligase (BOEHRINGER) et eau distillée q.s.p. 20μl.

La ligation et la transformation des bactéries *E. coli* compétentes sont effectuées comme décrit ci-dessus.

Les étapes de construction du plasmide pBγ1D7C2 sont représentées à la Figure 2.

La séquence de l'insert du plasmide pBγ1D7C2 qui code pour la chaine lourde gamma 1 d'un anticorps r-IgG-D7C2, et pour le peptide signal, est représentée dans la liste de séquence en annexe sous le numéro SEQ ID NO: 5 ; la séquence polypeptidique de la chaîne lourde gamma 1 codée par cet insert est représentée sous le numéro SEQ ID NO: 6.

**Exemple 3 : Construction d'un baculovirus recombinant produisant l'anticorps r-D7C2**

a - Insertion de la chaîne légère

Le plasmide chargé pBλD7C2 a été utilisé pour co-transfecter des cellules d'insecte avec de l'ADN du baculovirus sauvage AcMNPV.

Les conditions de transfection sont les suivantes : 500 ng d'ADN viral et 4 μg d'ADN plasmidique en présence de 40 μl de DOTAP (BOEHRINGER) dans 3 ml de milieu de culture sans sérum de veau pour cellules d'insectes. La cotransfection a été réalisée sur 4 x 10$^6$ cellules d'insectes Sf9 (ATCC35CRL 1711). Après quatre heures de contact à 28°C, le mélange de cotransfection est remplacé par 4 ml de milieu de TGV5 (milieu complet pour cellules d'insectes avec 5% de sérum de veau). La culture est continuée pendant 6 jours à 28°C.

Le virus produisant la chaîne légère de l'anticorps r-IgG-D7C2 sous le contrôle du promoteur polyédrine a été sélectionné dans un premier temps sur son incapacité à produire des polyèdres, puis sur sa capacité à exprimer une chaîne légère d'environ 25 kDa détectable par Western blot à l'aide d'anticorps anti-chaîne λ humaine du commerce (CALTAG, TEBU).

L'ADN du virus recombinant obtenu (appelé AcMNPV-λD7C2) a été préparé à partir des surnageants de cellules infectées.

b - Insertion de la chaîne lourde

Le plasmide chargé pBγ1D7C2 a été utilisé en cotransfection avec l'ADN du baculovirus modifié AcMNPV-λ D7C2. La cotransfection a été réalisée avec 500 ng d'ADN viral et 5 µg d'ADN plasmidique, sur cellules Sf9. Les doubles recombinants ont été sélectionnés par la technique de la dilution limite, associée à la technique ELISA.

Après une semaine de culture, le surnageant de cotransfection est dilué ($10^{-4}$ à $10^{-10}$) puis distribué en plaques de 96 puits sur des cellules Sf9 (100 µl de surnageant/puits), après une heure de contact, 100 µl de milieu sont rajoutés dans chaque puits. La sécrétion d'immunoglobulines γ1 est recherchée dans le surnageant de chaque puits par ELISA après une semaine de culture à 28°C.

Le test ELISA est réalisé comme indiqué ci-dessous :

50 µl de surnageant de culture de chaque puits sont déposés sur une plaque ELISA (NUNC) recouverte d'anticorps anti-IgG humaines totales (CALTAG, TEBU). L'adsorption des anticorps anti-IgG (100 µl d'une dilution 1/2000 par puits) a été réalisée en tampon PBS (137 mM NaCl ; 2,7 mM KCl ; 4,3 mM $Na_2HPO_4$ ; 1,4 mM $KH_2PO_4$) pendant une nuit à 4°C, après une saturation préalable pendant une heure à 37°C avec du PBS additionné de 5% de sérum de veau. Après l'ajout des surnageants de culture, 50 µl de PBS additionné de 1% de sérum de veau et 0,1% de Tween 20 sont rajoutés dans chaque puits. L'ensemble est incubé à 37°C et trois lavages en PBS additionné de 1% de Tween 20 sont effectués. Les plaques sont recouvertes d'anticorps anti-IgG humaines couplés à la biotine (CALTAG), à raison de 100 µl/puits d'une dilution 1/10 000 faite dans du PBS additionné de 1% de sérum de veau et 0,1% de Tween 20, pendant une heure à 37°C. Après trois nouveaux lavages, les plaques sont incubées en présence du conjugué streptavidine/phosphatase alcaline (dilution 1/10 000, CALTAG) puis révélées par le substrat p-nitrophényl-phosphate (1mg/ml, SIGMA) dilué en tampon alcalin (9 volumes de solution NaCl 3M et 1 volume de tampon Tris-HCl 1M pH 9,6). La densité optique de chaque puits est mesurée par spectrophotométrie à 405 nm. Les surnageants des puits positifs aux dilutions virales les plus fortes sont recueillis et conservés.

L'isolement d'un clone viral recombinant exprimant la chaîne légère et la chaîne lourde est assuré par deux autres séries d'infections en plaques de 96 puits et recherche de l'anticorps par ELISA, suivies d'un clonage par la technique des plages de lyse. Le virus sélectionné a été baptisé Ac10HRh-33LRh (313).

**Exemple 4 : Production et purification de l'anticorps monoclonal recombinant r-IgG-D7C2**

a) Culture et purification

Le virus sélectionné Ac10HRh-33LRh (313) est multiplié sur cellules d'insectes Sf9 en milieu TGV2 (2% de sérum de veau foetal). La concentration d'anticorps produit et sécrété est évaluée par ELISA ; elle est d'environ 3 mg/l.

Une fraction du surnageant viral obtenu est concentrée 10 fois à l'aide de concentrateurs CENTRIPREP 30 (AMICON), à une vitesse de rotation de 1800 tours par minute. Ce concentré est directement utilisé pour les tests d'activité biologique *in vitro*. La concentration d'anticorps recombinant est évaluée à 30 mg/l dans le concentré.

D'autre part, une fraction (50 ml) de surnageant viral est déposée sur une colonne de chromatographie de protéine A (gel SEPRACOR) préalablement équilibrée avec du PBS, afin de purifier l'IgG1 recombinante. Après deux rinçages successifs de la colonne avec du PBS et une solution citrate/acide citrique 0,1M pH5, l'immunoglobuline est: éluée avec une solution citrate/acide citrique 0,1M pH3. L'élution est suivie en mesurant la DO à 280 nm. Enfin, les fractions recueillies sont neutralisées à pH7 avec une solution de Tris-HCl pH8 et conservées à 4°C. La solution d'anticorps purifié est testée pour l'activité biologique parallèlement à la solution concentrée.

b) Contrôle de l'assemblage et de la taille des chaînes d'immunoglobuline

La qualité de l'anticorps produit par le virus recombinant Ac 10HRh-33LRh(313) a été contrôlée par migration électrophorétique dans un gel SDS-PAGE 12,5% en utilisant comme témoin une IgGλ humaine monoclonale du commerce (SIGMA). Cette expérience a montré que l'anticorps humain non réduit migre au même niveau que l'anticorps humain témoin. Après réduction par le dithiotréitol (DTT), on observe l'apparition de deux sous-unités correspondant aux chaînes lourdes et légères, et migrant au même niveau que les chaînes de l'anticorps humain contrôle traité de la même façon.

Pour confirmer ces résultats, les protéines ont été transférées sur une membrane de nitrocellulose, et les chaînes lourde et légère ont été détectées par des anticorps spécifiques anti-IgG ou anti-λ humaines.

**Exemple 5 : Activité biologique de l'anticorps monoclonal recombinant r-IgG-D7C2**

L'activité biologique de l'anticorps recombinant a été mesurée en utilisant le surnageant de culture des cellules d'insectes productrices de r-IgG-D7C2, concentré 10 fois. Les mesures ont été effectuées par rapport à un anticorps recombinant (IgG1,$\kappa$) "non-pertinent" (irrelevant) dirigé contre une protéine autre que l'antigène Rhésus (contrôle négatif) et produit dans les mêmes conditions en cellules d'insectes, et par rapport à l'anticorps monoclonal parental humain IgM-D7C2 (IgM,$\lambda$).

L'activité biologique de l'anticorps recombinant r-IgG-D7C2 a été évaluée :

1° Par des tests d'agglutination en tubes, avec le surnageant des cultures de cellules d'insectes concentré 10 fois (30$\mu$g/ml) et un panel de globules rouges humains papaïnés Rh-positif (R1/r, R1/R1, R2/R2, Ro/r), et Rh-négatif (r/r) (G.N.R.G.S.).

50 $\mu$l de surnageant de culture concentré, contenant r-IgG-D7C2, et 50 $\mu$l de la suspension à 2% de globules rouges ont été incubés pendant 45 minutes à 37° ; les agglutinations sont appréciées de + à +++ selon l'intensité de la réaction.

Les résultats figurent dans le Tableau I ci-dessous.

TABLEAU I

| GLOBULES ROUGES PAPAINES | R1/r | R1/R1 | R2/R2 | Ro/r | r/r |
|---|---|---|---|---|---|
| INTENSITE | + | + | +++ | ++ | - |

Le titre de l'anticorps recombinant r-IgG-D7C2, estimé en incubant le surnageant avec un pool de globules rouges R1/r, traité à la papaïne, pendant une heure à 37'C est de 1/512ème.

2° Par un test d'ADCC :

* Cellules effectrices (lymphocytes humains) :
La couche mononuclée est récupérée à partir du sang périphérique hépariné et séparé sur gradient de Ficoll Hypaque. Les cellules sont incubées à 37°, pendant une nuit, dans une boîte de culture cellulaire en plastique (pour supprimer les monocytes) avec 1% de sérum de veau foetal ; les cellules non adhérentes sont ensuite récupérées et utilisées pour le test de cytotoxicité à raison de $8 \times 10^6$ cellules/ml.

* Cellules cibles (globules rouges)

- Le sang veineux de donneurs normaux, de groupe R1/R1 (Rhésus positif) et de groupe r/r (Rhésus négatif), est prélevé sur citrate, puis les globules sont mis en suspension à 2% dans du NaCl et papaïnés.
- Marquage au chrome $Cr^{51}$
$20 \times 10^6$ globules rouges papaïnés sont incubés à 37° pendant une heure avec 200$\mu$Ci de $Cr^{51}$, lavés 4 fois et resuspendus dans du NaCl à 9 pour mille.
- Test ADCC

Les expériences sont effectuées en triple, incluant les combinaisons suivantes :

. globules rouges marqués au $Cr^{51}$ suspendus dans du NaCl à 9 pour mille, pour mesurer le relargage spontané du chrome.

. globules rouges marqués au $Cr^{51}$, suspendus dans de l'eau distillée pour mesurer le relargage maximum du chrome.

. globules rouges marqués au $Cr^{51}$ sans anticorps, pour mesurer la cytotoxicité éventuelle des cellules effectrices.

. globules rouges marqués au $Cr^{51}$, sensibilisés avec :

- l'anti-D recombinant r-IgG-D7C2 ;
- un anticorps anti-D polyclonal spécifique (gammaglobulines, CNTS) à titre de contrôle positif ;
- l'anticorps recombinant "non-pertinent", à titre de contrôle négatif.

Les anticorps sont utilisés à deux concentrations différentes (7,5$\mu$g/ml, 3.5$\mu$g/ml).

Les cellules préparées, effectrices et cibles sont distribuées dans des microplaques à fond rond de la manière suivante :

50 $\mu$l de lymphocytes ($8.10^6$ cellules/ml) et 50$\mu$l de la suspension de globules rouges marqués au $Cr^{51}$ ($8.10^5$ cellules/ml) sont mis dans chaque puits avec un ratio de 10/1 ; 50$\mu$l des différents anticorps sont ajoutés ensuite pour l'étude comparative. La quantité de $Cr^{51}$ relargué est mesurée pour chaque suspension de globules rouges, et le % de lyse spécifique est calculé selon la formule suivante :

$$\frac{(\% \text{ relargage d'un test}) - (\% \text{ relargage spontané})}{(\% \text{ relargage maximum}) - (\% \text{ relargage spontané})} \times 100$$

Les résultats figurent sur les tableaux II (hématies R1/R1) et III (hématies r/r)ci-dessous.

TABLEAU II

|  | Concentration d'anticorps | % de Lyse spécifique |
|---|---|---|
| Lymphocytes + Hématies |  | 0,65% |
| Lymphocytes + Hématies + Anticorps témoin (IgG1) recombinant | 3,5 µg/ml | 0% |
| Lymphocytes + Hématies + r-IgG-D7C2 | 7,5 µg/ml | 76,6% |
|  | 3,5 µg/ml | 93,3% |
| Lymphocytes + Hématies + Anti-D polyclonal (γ globulines) | 7,5 µg/ml | 66,9% |
|  | 3,5 µg/ml | 76,0% |

TABLEAU III

|  | Concentration d'anticorps | % de Lyse spécifique |
|---|---|---|
| Lymphocytes + Hématies |  | 0,76% |
| Lymphocytes + Hématies + Anticorps témoin (IgG1) recombinant | 3,5 µg/ml | 0% |
| Lymphocytes + Hématies + r-IgG-D7C2 | 7,5 µg/ml | 1,3% |
|  | 3,5 µg/ml | 0% |
| Lymphocytes + Hématies + Anti-D polyclonal (γ globulines) | 7,5 µg/ml | 0% |
|  | 3,5 µg/ml | 0,1% |

Ces résultats montrent que l'anticorps r-IgG-D7C2 induit une lyse spécifique des hématies Rh-positif R1/R1.

**Revendications**

1. Fragment d'ADN, **caractérisé, en ce qu'**il est choisi dans le groupe constitué par :

- un fragment d'ADN qui code pour le domaine variable de la chaîne légère de l'anticorps monoclonal D7C2, ledit domaine variable étant défini par la séquence SEQ ID : 2 ;
- un fragment d'ADN qui code pour le domaine variable de la chaîne lourde de l'anticorps monoclonal D7C2, ledit domaine variable étant défini par la séquence SEQ ID NO : 4.

2. Cassette d'expression **caractérisée en ce qu'**elle comprend au moins un fragment d'ADN selon la revendication 1, placé sous contrôle transcriptionnel d'un promoteur de baculovirus.

3. Cassette d'expression selon la revendication 2, **caractérisée en ce qu'**elle comprend les éléments permettant l'expression de la chaîne lourde ou de la chaîne légère d'un anticorps monoclonal recombinant dénommé r-D7C2, lesdits éléments étant constitués par :

- un promoteur de baculovirus, sous contrôle transcriptionnel duquel sont placés :

    - une séquence codant pour un peptide signal de sécrétion ; et
    - une séquence codant pour le domaine variable de la chaîne légère de l'anticorps monoclonal D7C2 et une séquence codant pour le domaine constant de la chaîne légère d'une immunoglobuline ; ou bien
    - une séquence codant pour le domaine variable de la chaîne lourde de l'anticorps monoclonal D7C2 et une séquence codant pour le domaine constant de la chaîne lourde d'une immunoglobuline.

4. Cassette d'expression selon la revendication 2, **caractérisée en ce que** la séquence codant pour le domaine constant de la chaîne légère ou de la chaîne lourde d'immunoglobuline est une séquence d'origine humaine.

5. Cassette d'expression selon une quelconque des revendications 2 à 4, **caractérisée en ce que** le promoteur de baculovirus est le promoteur de la polyédrine ou l'un de ses dérivés, ou bien le promoteur de la p10 ou l'un de ses dérivés.

6. Vecteur recombinant, **caractérisé en ce qu'**il comprend au moins un fragment d'ADN selon la revendication 1.

7. Vecteur recombinant selon la revendication 6, **caractérisé en ce qu'**il comprend au moins une cassette d'expression selon une quelconque des revendications 2 à 5.

8. Vecteur recombinant selon la revendication 7, **caractérisé en ce qu'**il constitue un vecteur de transfert, portant un insert comprenant : une cassette d'expression selon une quelconque des revendications 2 à 4, et de part et d'autre de cette cassette, des séquences de baculovirus homologues de celles des régions flanquant la portion du génome de baculovirus en remplacement de laquelle on souhaite insérer ladite cassette.

9. Vecteur de transfert selon la revendication 8, **caractérisé en ce que** lesdites séquences de baculovirus sont homologues de celles des régions flanquant le gène de la p10, ou homologues de celles des régions flanquant le gène de la polyédrine.

10. Vecteur de transfert selon la revendication 9, **caractérisé en ce que** la cassette d'expression contenant le gène codant pour la chaîne légère de l'anticorps r-D7C2 est flanquée des régions entourant le gène de la polyédrine dans le baculovirus sauvage, et la cassette d'expression portant le gène codant pour la chaîne lourde de l'anticorps r-D7C2 est flanquée des régions entourant le gène P10 dans le baculovirus sauvage.

11. Baculovirus recombinant **caractérisé en ce qu'**il comprend au moins une cassette d'expression selon une quelconque des revendications 2 à 5.

12. Baculovirus recombinant selon la revendication 11, **caractérisé en ce qu'**il comprend une cassette d'expression comprenant la séquence codant pour la chaîne H de l'anticorps r-D7C2, et une cassette d'expression comprenant la séquence codant pour la chaîne L de l'anticorps r-D7C2.

13. Baculovirus recombinant selon la revendication 12, **caractérisé en ce que** le promoteur contrôlant la transcription de la séquence codant pour la chaîne L de l'anticorps monoclonal r-D7C2, et le promoteur contrôlant la transcription de la séquence codant pour la chaîne H de l'anticorps monoclonal r-D7C2, sont deux promoteurs différents.

14. Baculovirus recombinant selon la revendication 13, **caractérisé en ce que** l'un desdits promoteurs est situé à l'emplacement occupé chez le baculovirus sauvage, par le promoteur de la polyédrine et l'autre est situé à l'emplacement occupé chez le baculovirus sauvage, par le promoteur de la P10.

15. Baculovirus recombinant selon une quelconque des revendications 11 à 14, **caractérisé en ce que** la transcription de la séquence codant pour la chaîne légère de l'anticorps r-D7C2 est sous contrôle du promoteur de la polyédrine ou de l'un de ses dérivés, et la transcription de la séquence codant pour la chaîne lourde de l'anticorps r-D7C2 est sous contrôle du promoteur de la P10 ou de l'un de ses dérivés.

16. Baculovirus recombinant selon une quelconque des revendications 12 à 14, **caractérisé en ce que** la séquence codant pour le peptide signal associé à la chaîne L de l'anticorps monoclonal r-D7C2, et la séquence codant pour le peptide signal associé à la chaîne H de l'anticorps monoclonal r-D7C2, sont deux séquences différentes.

17. Baculovirus recombinant selon la revendication 16, déposé le 19 août 1994, auprès de la C.N.C.M., sous le numéro I-1468.

18. Cellules d'insecte infectées avec un baculovirus recombinant selon une quelconque des revendications 11 à 17.

19. Procédé de préparation d'un anticorps monoclonal recombinant, **caractérisé en ce qu'**il comprend une étape au cours de laquelle l'on cultive des cellules d'insecte selon la revendication 18, et une étape au cours de laquelle l'on obtient ledit anticorps à partir du milieu de culture.

20. Anticorps monoclonal recombinant, **caractérisé en ce que** ses domaines variables sont constitués par :

   - le domaine variable de la chaîne légère de l'anticorps monoclonal D7C2, ledit domaine variable étant défini par la séquence SEQ ID NO : 2 ; et
   - le domaine variable de la chaîne lourde de l'anticorps monoclonal D7C2, ledit domaine variable étant défini par la séquence SEQ ID NO : 4.

21. Anticorps monoclonal recombinant selon la revendication 20, **caractérisé en ce qu'**il est susceptible d'être obtenu par un procédé selon la revendication 19.

22. Anticorps monoclonal recombinant selon une quelconque des revendications 20 ou 21 **caractérisé en ce que** la séquence polypeptidique de sa chaîne légère et la séquence polypeptidique de sa chaîne lourde sont des séquences humaines.

**23.** Anticorps monoclonal recombinant selon une quelconque des revendications 20 à 22 **caractérisé en ce qu'**il appartient à la classe des IgG.

**24.** Utilisation d'un anticorps monoclonal recombinant selon une quelconque des revendications 20 à 23 pour l'obtention de médicaments.

**Patentansprüche**

**1.** DNA-Fragment, **dadurch gekennzeichnet, dass** es aus der Gruppe ausgewählt ist bestehend aus:

- einem DNA-Fragment, das für die variable Domäne der leichten Kette des monoklonalen Antikörpers D7C2 kodiert, wobei diese variable Domäne durch die Sequenz SEQ ID NO: 2 definiert ist;

- einem DNA-Fragment, das für die variable Domäne der schweren Kette des monoklonalen Antikörpers D7C2 kodiert, wobei diese variable Domäne durch die Sequenz SEQ ID NO: 4 definiert ist.

**2.** Expressionskassette, **dadurch gekennzeichnet, dass** sie wenigstens ein DNA-Fragment nach Anspruch 1 unter transkriptioneller Kontrolle eines Baculovirus-Promotors umfasst.

**3.** Expressionskassette nach Anspruch 2, **dadurch gekennzeichnet, dass** sie die Elemente umfasst, die die Expression der schweren Kette oder der leichten Kette eines als r-D7C2 bezeichneten rekombinanten monoklonalen Antikörpers erlauben, wobei diese Elemente bestehen aus:

- einem Baculovirus-Promotor, unter dessen transkriptioneller Kontrolle stehen:

    - eine Sequenz, die für ein Signalpeptid für die Sekretion kodiert; und

    - eine Sequenz, die für die variable Domäne der leichten Kette des monoklonalen Antikörpers D7C2 kodiert, und eine Sequenz, die für die konstante Domäne der leichten Kette eines Immunglobulins kodiert; oder

    - eine Sequenz, die für die variable Domäne der schweren Kette des monoklonalen Antikörpers D7C2 kodiert, und eine Sequenz, die für die konstante Domäne der schweren Kette eines Immunglobulins kodiert.

**4.** Expressionskassette nach Anspruch 2, **dadurch gekennzeichnet, dass** die Sequenz, die für die konstante Domäne der leichten oder der schweren Immunglobulinkette kodiert, eine Sequenz humanen Ursprungs ist.

**5.** Expressionskassette nach irgendeinem der Ansprüche 2 bis 4, **dadurch gekennzeichnet, dass** der Baculovirus-Promotor der Promotor für Polyhedrin oder eines seiner Derivate oder der Promotor für p10 oder eines seiner Derivate ist.

**6.** Rekombinanter Vektor, **dadurch gekennzeichnet, dass** er wenigstens ein DNA-Fragment nach Anspruch 1 umfasst.

**7.** Rekombinanter Vektor nach Anspruch 6, **dadurch gekennzeichnet, dass** er wenigstens eine Expressionskassette nach irgendeinem der Ansprüche 2 bis 5 umfasst.

**8.** Rekombinanter Vektor nach Anspruch 7, **dadurch gekennzeichnet, dass** er einen Transfervektor darstellt, der ein Insert trägt, das umfasst: eine Expressionskassette nach irgendeinem der Ansprüche 2 bis 4 und zu beiden Seiten dieser Kassette Baculovirus-Sequenzen, die homolog zu denen der Regionen sind, die den Teil des Baculovirus-Genoms flankieren, an dessen Stelle die Kassette inseriert werden soll.

**9.** Transfervektor nach Anspruch 8, **dadurch gekennzeichnet, dass** diese Baculovirus-Sequenzen homolog zu denen der Regionen sind, die das Gen für p10 flankieren, oder homolog zu denen der Regionen, die das Gen für Polyhedrin flankieren.

**10.** Transfervektor nach Anspruch 9, **dadurch gekennzeichnet, dass** die Expressionskassette, die das Gen enthält, das für die leichte Kette des Antikörpers r-D7C2 kodiert, von den Regionen flankiert wird, die das Gen für Polyhedrin in Wildtyp-Baculovirus einschließen, und die Expressionskassette, die das Gen trägt, das für die schwere Kette des Antikörpers r-D7C2 kodiert, von den Regionen flankiert wird, die das P10-Gen in Wildtyp-Baculovirus einschließen.

11. Rekombinantes Baculovirus, **dadurch gekennzeichnet, dass** es wenigstens eine Expressionskassette nach irgendeinem der Ansprüche 2 bis 5 umfasst.

12. Rekombinantes Baculovirus nach Anspruch 11, **dadurch gekennzeichnet, dass** es eine Expressionskassette umfasst, die die Sequenz umfasst, die für die H-Kette des Antikörpers r-D7C2 kodiert, und eine Expressionskassette, die die Sequenz umfasst, die für die L-Kette des Antikörpers r-D7C2 kodiert.

13. Rekombinantes Baculovirus nach Anspruch 12, **dadurch gekennzeichnet, dass** der Promotor, der die Transkription der Sequenz kontrolliert, die für die L-Kette des monoklonalen Antikörpers r-D7C2 kodiert, und der Promotor, der die Transkription der Sequenz kontrolliert, die für die H-Kette des monoklonalen Antikörpers r-D7C2 kodiert, zwei unterschiedliche Promotoren sind.

14. Rekombinantes Baculovirus nach Anspruch 13, **dadurch gekennzeichnet, dass** sich der eine dieser Promotoren an der Stelle befindet, die bei Wildtyp-Baculovirus durch den Promotor für Polyhedrin ausgefüllt ist, und sich der andere an der Stelle befindet, die bei Wildtyp-Baculovirus durch den Promotor für P10 ausgefüllt ist.

15. Rekombinantes Baculovirus nach irgendeinem der Ansprüche 11 bis 14, **dadurch gekennzeichnet, dass** die Transkription der Sequenz, die für die leichte Kette des Antikörpers r-D7C2 kodiert, unter Kontrolle des Promotors für Polyhedrin oder einem seiner Derivate steht, und die Transkription der Sequenz, die für die schwere Kette des Antikörpers r-D7C2 kodiert, unter Kontrolle des Promotors für P10 oder einem seiner Derivate steht.

16. Rekombinantes Baculovirus nach irgendeinem der Ansprüche 12 bis 14, **dadurch gekennzeichnet, dass** die Sequenz, die für das Signalpeptid kodiert, das mit der L-Kette des monoklonalen Antikörpers r-D7C2 verbunden ist, und die Sequenz, die für das Signalpeptid kodiert, das mit der H-Kette des monoklonalen Antikörpers r-D7C2 verbunden ist, zwei unterschiedliche Sequenzen sind.

17. Rekombinantes Baculovirus nach Anspruch 16, hinterlegt bei der C.N.C.M. am 19. August 1994 unter der Nummer I-1468.

18. Insektenzellen, die mit einem rekombinanten Baculovirus nach irgendeinem der Ansprüche 11 bis 17 infiziert sind.

19. Verfahren zur Herstellung eines rekombinanten monoklonalen Antikörpers, **dadurch gekennzeichnet, dass** es einen Schritt umfasst, bei dem man Insektenzellen nach Anspruch 18 kultiviert, und einen Schritt, bei dem man den Antikörper aus dem Kulturmedium erhält.

20. Rekombinanter monoklonaler Antikörper, **dadurch gekennzeichnet, dass** seine variablen Domänen bestehen aus:

   - der variablen Domäne der leichten Kette des monoklonalen Antikörpers D7C2, wobei diese variable Domäne durch die Sequenz SEQ ID NO: 2 definiert ist; und

   - der variablen Domäne der schweren Kette des monoklonalen Antikörpers D7C2, wobei diese variable Domäne durch die Sequenz SEQ ID NO: 4 definiert ist.

21. Rekombinanter monoklonaler Antikörper nach Anspruch 20, **dadurch gekennzeichnet, dass** er nach einem Verfahren nach Anspruch 19 erhältlich ist.

22. Rekombinanter monoklonaler Antikörper nach irgendeinem der Ansprüche 20 oder 21, **dadurch gekennzeichnet, dass** die Polypeptidsequenz seiner leichten Kette und die Polypeptidsequenz seiner schweren Kette humane Sequenzen sind.

23. Rekombinanter monoklonaler Antikörper nach irgendeinem der Ansprüche 20 bis 22, **dadurch gekennzeichnet, dass** er Zur Klasse der IgG gehört.

# EP 0 778 891 B1

**24.** Verwendung eines rekombinanten monoklonalen Antikörpers nach irgendeinem der Ansprüche 20 bis 23 zur Herstellung von Medikamenten.

**Claims**

**1.** DNA fragment, **characterized in that** it is chosen from the group consisting of:

- a DNA fragment which encodes the variable domain of the light chain of the D7C2 monoclonal antibody, said variable domain is defined by the sequence SEQ ID : 2;
- a DNA fragment which encodes the variable domain of the heavy chain of the D7C2 monoclonal antibody, said variable domain is defined by the sequence SEQ ID : 4.

**2.** Expression cassette, **characterized in that** it comprises at least one DNA fragment according to Claim 1, placed under the transcriptional control of a baculovirus promoter.

**3.** Expression cassette according to Claim 2, **characterized in that** it comprises the elements allowing the expression of the heavy chain or of the light chain of a recombinant monoclonal antibody called r-D7C2, the said elements consisting of:

- a baculovirus promoter, under whose transcriptional control are placed:

- a sequence encoding a secretory signal peptide; and
- a sequence encoding the variable domain of the light chain of the D7C2 monoclonal antibody and a sequence encoding the constant domain of the light chain of an immunoglobulin; or alternatively
- a sequence encoding the variable domain of the heavy chain of the D7C2 monoclonal antibody and a sequence encoding the constant domain of the heavy chain of an immunoglobulin.

**4.** Expression cassette according to Claim 2, **characterized in that** the sequence encoding the constant domain of the light chain or of the heavy chain of an immunoglobulin is a sequence of human origin.

**5.** Expression cassette according to any one of Claims 2 to 4, **characterized in that** the baculovirus promoter is the polyhedrin promoter or one of its derivatives, or alternatively the p10 promoter or one of its derivatives.

**6.** Recombinant vector, **characterized in that** it comprises at least one DNA fragment according to Claim 1.

**7.** Recombinant vector according to Claim 6, **characterized in that** it comprises at least one expression cassette according to any one of Claims 2 to 5.

**8.** Recombinant vector according to Claim 7, **characterized in that** it constitutes a transfer vector, carrying an insert comprising: an expression cassette according to any one of Claims 2 to 4, and on either side of this cassette, baculovirus sequences homologous to those of the regions flanking the portion of the baculovirus genome for whose replacement it is desired to insert the said cassette.

**9.** Transfer vector according to Claim 8, **characterized in that** the said baculovirus sequences are homologous to those of the regions flanking the p10 gene, or homologous to those of the regions flanking the polyhedrin gene.

**10.** Transfer vector according to Claim 9, **characterized in that** the expression cassette containing the gene encoding the light chain of the r-D7C2 antibody is flanked by regions surrounding the polyhedrin gene in the wild-type baculovirus, and the expression cassette carrying the gene encoding the heavy chain of the r-D7C2 antibody is flanked by the regions surrounding the P10 gene in the wild-type baculovirus.

**11.** Recombinant baculovirus **characterized in that** it comprises at least one expression cassette according to any one of Claims 2 to 5.

**12.** Recombinant baculovirus according to Claim 11, **characterized in that** it comprises an expression cassette comprising the sequence encoding the H chain of the r-D7C2 antibody, and an expression cassette comprising the sequence encoding the L chain of the r-D7C2 antibody.

**34**

**13.** Recombinant baculovirus according to Claim 12, **characterized in that** the promoter controlling the transcription of the sequence encoding the L chain of the r-D7C2 monoclonal antibody, and the promoter controlling the transcription of the sequence encoding the H chain of the r-D7C2 monoclonal antibody, are two different promoters.

**14.** Recombinant baculovirus according to Claim 13, **characterized in that** one of the said promoters is situated at the site occupied in the wild-type baculovirus by the polyhedrin promoter and the other is situated at the site occupied in the wild-type baculovirus by the P10 promoter.

**15.** Recombinant baculovirus according to any one of Claims 11 to 14, **characterized in that** the transcription of the sequence encoding the light chain of the r-D7C2 antibody is under the control of the polyhedrin promoter or of one of its derivatives, and the transcription of the sequence encoding the heavy chain of the r-D7C2 antibody is under the control of the P10 promoter or of one of its derivatives.

**16.** Recombinant baculovirus according to any one of Claims 12 to 14, **characterized in that** the sequence encoding the signal peptide associated with the L chain of the r-D7C2 monoclonal antibody, and the sequence encoding the signal peptide associated with the H chain of the r-D7C2 monoclonal antibody, are two different sequences.

**17.** Recombinant baculovirus according to Claim 16, deposited on 19 August 1994, at the C.N.C.M., under the number I-1468.

**18.** Insect cells infected with a recombinant baculovirus according to any one of Claims 11 to 17.

**19.** Process for the preparation of a recombinant monoclonal antibody, **characterized in that** it comprises a stage during which insect cells according to Claim 18 are cultured, and a stage during which the said antibody is obtained from the culture medium.

**20.** Recombinant monoclonal antibody, **characterized in that** its variable domains consisting of:

- the variable domain of the light chain of the D7C2 monoclonal antibody, said variable domain is defined by the sequence SEQ ID : 2;
- the variable domain of the heavy chain of the D7C2 monoclonal antibody, said variable domain is defined by the sequence SEQ ID : 4.

**21.** Recombinant monoclonal antibody according to Claim 20, **characterized in that** it is capable of being obtained by a process according to Claim 19.

**22.** Recombinant monoclonal antibody according to either of Claims 20 and 21, **characterized in that** the polypeptide sequence of its light chain and the polypeptide sequence of its heavy chain are human sequences.

**23.** Recombinant monoclonal antibody according to any one of Claims 20 to 22, **characterized in that** it belongs to the IgG class.

**24.** Use of a recombinant monoclonal antibody according to any one of Claims 20 to 23, for the production of medicaments.

## FIGURE 1

## FIGURE 2

LISTE DE SEQUENCES

(1) INFORMATIONS GENERALES:

(i) DEPOSANT:

(A) NOM: INSTITUT PASTEUR
(B) RUE: 28, RUE DU DOCTEUR ROUX
(C) VILLE: PARIS
(D) ETAT OU PROVINCE: -
(E) PAYS: FRANCE
(F) CODE POSTAL: 75724 CEDEX 15

(A) NOM: PROTEINE PERFORMANCE - SOCIETE ANONYME
(B) RUE: ROUTE D'ALES
(C) VILLE: SAINT CHRISTOL-LES-ALES
(D) ETAT OU PROVINCE: -
(E) PAYS: FRANCE

(F) CODE POSTAL: 30380

(A) NOM: EDELMAN LENA
(B) RUE: 6 RUE FESSART
(C) VILLE: BOULOGNE
(D) ETAT OU PROVINCE: -
(E) PAYS: FRANCE
(F) CODE POSTAL: 92100

(A) NOM: MARGARITTE CHRISTEL
(B) RUE: 16 RUE CHARLES DE GAULLE
(C) VILLE: CHATENAY-MALABRY
(D) ETAT OU PROVINCE: -
(E) PAYS: FRANCE
(F) CODE POSTAL: 92290

(A) NOM: KACZORECK MICHEL
(B) RUE: 81 BOULEVARD DE LA LIRONDE
(C) VILLE: MONTFERRIER
(D) ETAT OU PROVINCE: -
(E) PAYS: FRANCE
(F) CODE POSTAL: 34980

(A) NOM: CHAABIHI HASSAN
(B) RUE: 30B AVENUE JULES GUESDE
(C) VILLE: ALES
(D) ETAT OU PROVINCE: -
(E) PAYS: FRANCE
(F) CODE POSTAL: 30100

(ii) TITRE DE L' INVENTION: OBTENTION D'UN ANTICORPS MONOCLONAL RECOMBINANT A PARTIR D'UN ANTICORPS MONOCLONAL HUMAIN ANTI-RHESUS D, SA PRODUCTION EN CELLULES D'INSECTE, ET SES UTILISATIONS.

(iii) NOMBRE DE SEQUENCES: 25

(iv) FORME DECHIFFRABLE PAR ORDINATEUR:

(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.30 (OEB)

(2) INFORMATIONS POUR LA SEQ ID NO: 1:

(i) CARACTERISTIQUES DE LA SEQUENCE:

(A) LONGUEUR: 312 paires de bases
(B) TYPE: nucléotide
(C) NOMBRE DE BRINS: simple
(D) CONFIGURATION: linéaire

(ix) CARACTERISTIQUE:

(A) NOM/CLE: CDS
(B) EMPLACEMENT:1..312
(D) AUTRES INFORMATIONS:/product= "Immunoglobulin Variable Region"

(ix) CARACTERISTIQUE:

    (A) NOM/CLE: misc_feature
    (B) EMPLACEMENT:67..99
    (D) AUTRES INFORMATIONS:/label= CDR1

(ix) CARACTERISTIQUE:

    (A) NOM/CLE: misc_feature
    (B) EMPLACEMENT:145..165
    (D) AUTRES INFORMATIONS:/label= CDR2

(ix) CARACTERISTIQUE:

    (A) NOM/CLE: misc_feature
    (B) EMPLACEMENT:262..279
    (D) AUTRES INFORMATIONS:/label= CDR3

(xi) DESCRIPTION DE LA SEQUENCE: SEQ ID NO: 1:

```
GAC ATC GAG CTC ACT CAG GAC CCT GCT GTG TCT GTG GCC TTG GGA CAG      48
Asp Ile Glu Leu Thr Gln Asp Pro Ala Val Ser Val Ala Leu Gly Gln
 1               5                  10                  15

ACA GTC AGG ATC ACA TGC CAA GGA GAC AGC CTC AGA ACC TAT TAT GCA      96
Thr Val Arg Ile Thr Cys Gln Gly Asp Ser Leu Arg Thr Tyr Tyr Ala
             20                  25                  30

AGC TGG TAC CAG CAG AAG CCA GGA CAG GCA CCT GTA CTT GTC ATC TAT     144
Ser Trp Tyr Gln Gln Lys Pro Gly Gln Ala Pro Val Leu Val Ile Tyr
         35                  40                  45

GGT AAA AAC AAC CGG CCC TCA GGG ATC CCA GAC CGA TTC TCT GGC TCC     192
Gly Lys Asn Asn Arg Pro Ser Gly Ile Pro Asp Arg Phe Ser Gly Ser
     50                  55                  60

AGC TCA GGA AAC ACA GCT TCC TTG ACC ATC ACT GGG GCT CAG GCG GAA     240
Ser Ser Gly Asn Thr Ala Ser Leu Thr Ile Thr Gly Ala Gln Ala Glu
 65                  70                  75                  80

GAT GAG GCT GAC TAT TTC TGT AAC AGC GGT GGG AAG GTG TTC GGC GGA     288
Asp Glu Ala Asp Tyr Phe Cys Asn Ser Gly Gly Lys Val Phe Gly Gly
                 85                  90                  95

GGG ACC AAG CTG ACC GTC CTA GGT                                     312
Gly Thr Lys Leu Thr Val Leu Gly
                100
```

(2) INFORMATIONS POUR LA SEQ ID NO: 2:

    (i) CARACTERISTIQUES DE LA SEQUENCE:

        (A) LONGUEUR: 104 acides aminés
        (B) TYPE: acide aminé
        (D) CONFIGURATION: linéaire

    (ii) TYPE DE MOLECULE: protéine

    (xi) DESCRIPTION DE LA SEQUENCE: SEQ ID NO: 2:

```
Asp Ile Glu Leu Thr Gln Asp Pro Ala Val Ser Val Ala Leu Gly Gln
 1               5                   10                  15

Thr Val Arg Ile Thr Cys Gln Gly Asp Ser Leu Arg Thr Tyr Tyr Ala
            20              25                  30

Ser Trp Tyr Gln Gln Lys Pro Gly Gln Ala Pro Val Leu Val Ile Tyr
        35                  40                  45

Gly Lys Asn Asn Arg Pro Ser Gly Ile Pro Asp Arg Phe Ser Gly Ser
        50                  55                  60

Ser Ser Gly Asn Thr Ala Ser Leu Thr Ile Thr Gly Ala Gln Ala Glu
 65                 70                  75                  80

Asp Glu Ala Asp Tyr Phe Cys Asn Ser Gly Gly Lys Val Phe Gly Gly
                85                  90                  95

Gly Thr Lys Leu Thr Val Leu Gly
                100
```

(2) INFORMATIONS POUR LA SEQ ID NO: 3:

   (i) CARACTERISTIQUES DE LA SEQUENCE:

      (A) LONGUEUR: 369 paires de bases
      (B) TYPE: nucléotide
      (C) NOMBRE DE BRINS: simple
      (D) CONFIGURATION: linéaire

   (ix) CARACTERISTIQUE:

      (A) NOM/CLE: CDS
      (B) EMPLACEMENT:1..369
      (D) AUTRES INFORMATIONS:/product= "Immunoglobulin Variable Region"

   (ix) CARACTERISTIQUE:

      (A) NOM/CLE: misc_feature
      (B) EMPLACEMENT:91..105
      (D) AUTRES INFORMATIONS:/label= CDR1

   (ix) CARACTERISTIQUE:

      (A) NOM/CLE: misc_feature
      (B) EMPLACEMENT:148..195
      (D) AUTRES INFORMATIONS:/label= CDR2

   (ix) CARACTERISTIQUE:

      (A) NOM/CLE: misc_feature
      (B) EMPLACEMENT:292..336
      (D) AUTRES INFORMATIONS:/label= CDR3

(xi) DESCRIPTION DE LA SEQUENCE: SEQ ID NO: 3:

```
CAG GTC CAA CTG CAG CAG TGG GGC GCA GGA CTG TTG AAG CCT TCG GAG        48
Gln Val Gln Leu Gln Gln Trp Gly Ala Gly Leu Leu Lys Pro Ser Glu
105             110             115             120

ACC CTG TCC CTC ACC TGC ACT GTC TAT GGT GGG TCC TTC AGT GGT TAC        96
Thr Leu Ser Leu Thr Cys Thr Val Tyr Gly Gly Ser Phe Ser Gly Tyr
            125             130             135

TAC TGG AGC TGG ATC CGC CAG CCC CCA GGG AAG GGG CTG GAG TGG ATT       144
Tyr Trp Ser Trp Ile Arg Gln Pro Pro Gly Lys Gly Leu Glu Trp Ile
            140             145             150

GGG GAA ATC AAT CAT AGT GGA AGC ACC AAC TAC AAC CCG TCC CTC AAG       192
Gly Glu Ile Asn His Ser Gly Ser Thr Asn Tyr Asn Pro Ser Leu Lys
            155             160             165

AGT CGA GTC ACC ATA TCA GTA GAC ACG TCC AAG AAC CAG TTC TCC CTG       240
Ser Arg Val Thr Ile Ser Val Asp Thr Ser Lys Asn Gln Phe Ser Leu
        170             175             180

AAA CTG AAC TCT GTG ACC GCC GCG GAC ACG GCT GTG TAT TAC TGT GCG       288
Lys Leu Asn Ser Val Thr Ala Ala Asp Thr Ala Val Tyr Tyr Cys Ala
185             190             195             200
AGG GCC CCA GAG TAT AAA TGG AAG TAT CAT GGG GAC TGG TTC GAC CCC       336
Arg Ala Pro Glu Tyr Lys Trp Lys Tyr His Gly Asp Trp Phe Asp Pro
            205             210             215

TGG GGC CAA GGT ACC ACT GTC ACC GTC TCC TCA                          369
Trp Gly Gln Gly Thr Thr Val Thr Val Ser Ser
            220             225
```

(2) INFORMATIONS POUR LA SEQ ID NO: 4:

    (i) CARACTERISTIQUES DE LA SEQUENCE:

        (A) LONGUEUR: 123 acides aminés
        (B) TYPE: acide aminé
        (D) CONFIGURATION: linéaire

    (ii) TYPE DE MOLECULE: protéine

    (xi) DESCRIPTION DE LA SEQUENCE: SEQ ID NO: 4:

```
Gln Val Gln Leu Gln Gln Trp Gly Ala Gly Leu Leu Lys Pro Ser Glu
 1               5                   10                  15

Thr Leu Ser Leu Thr Cys Thr Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asn His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Asn Ser Val Thr Ala Ala Asp Thr Ala Val Tyr Tyr Cys Ala
            85                  90                  95
```

```
Arg Ala Pro Glu Tyr Lys Trp Lys Tyr His Gly Asp Trp Phe Asp Pro
            100                 105                 110

Trp Gly Gln Gly Thr Thr Val Thr Val Ser Ser
        115                 120
```

(2) INFORMATIONS POUR LA SEQ ID NO: 5:

    (i) CARACTERISTIQUES DE LA SEQUENCE:

        (A) LONGUEUR: 716 paires de bases
        (B) TYPE: nucléotide
        (C) NOMBRE DE BRINS: simple
        (D) CONFIGURATION: linéaire

    (ix) CARACTERISTIQUE:

        (A) NOM/CLE: CDS
        (B) EMPLACEMENT:1..716

    (ix) CARACTERISTIQUE:

        (A) NOM/CLE: sig_peptide
        (B) EMPLACEMENT:1..57

    (ix) CARACTERISTIQUE:

        (A) NOM/CLE: mat_peptide
        (B) EMPLACEMENT:58..716
        (D) AUTRES INFORMATIONS:/product= "Immunoglobulin, Light Chain"

    (xi) DESCRIPTION DE LA SEQUENCE: SEQ ID NO: 5:

```
ATG GGA TGG AGC TGT ATC ATC CTC TTC TTG GTA GCA ACA GCT ACA GGT     48
Met Gly Trp Ser Cys Ile Ile Leu Phe Leu Val Ala Thr Ala Thr Gly
-19             -15             -10                     -5

GTC CAC TCC GAC ATC GAG CTC ACT CAG GAC CCT GCT GTG TCT GTG GCC     96
Val His Ser Asp Ile Glu Leu Thr Gln Asp Pro Ala Val Ser Val Ala
            1               5                   10

TTG GGA CAG ACA GTC AGG ATC ACA TGC CAA GGA GAC AGC CTC AGA ACC    144
Leu Gly Gln Thr Val Arg Ile Thr Cys Gln Gly Asp Ser Leu Arg Thr
        15              20              25

TAT TAT GCA AGC TGG TAC CAG CAG AAG CCA GGA CAG GCA CCT GTA CTT    192
Tyr Tyr Ala Ser Trp Tyr Gln Gln Lys Pro Gly Gln Ala Pro Val Leu
30              35                  40                  45

GTC ATC TAT GGT AAA AAC AAC CGG CCC TCA GGG ATC CCA GAC CGA TTC    240
Val Ile Tyr Gly Lys Asn Asn Arg Pro Ser Gly Ile Pro Asp Arg Phe
            50                  55                  60

TCT GGC TCC AGC TCA GGA AAC ACA GCT TCC TTG ACC ATC ACT GGG GCT    288
Ser Gly Ser Ser Ser Gly Asn Thr Ala Ser Leu Thr Ile Thr Gly Ala
            65                  70                  75

CAG GCG GAA GAT GAG GCT GAC TAT TTC TGT AAC AGC GGT GGG AAG GTG    336
Gln Ala Glu Asp Glu Ala Asp Tyr Phe Cys Asn Ser Gly Gly Lys Val
        80                  85                  90

TTC GGC GGA GGG ACC AAG CTG ACC GTC CTA GGT CAG CCC AAG GCT GCC    384
Phe Gly Gly Gly Thr Lys Leu Thr Val Leu Gly Gln Pro Lys Ala Ala
        95                  100                 105

CCC TCG GTC ACT CTG TTC CCG CCC TCC CTC GAG GAG CTT CAA GCC AAC    432
Pro Ser Val Thr Leu Phe Pro Pro Ser Leu Glu Glu Leu Gln Ala Asn
110                 115                 120                 125

AAG GCC ACA CTC GAG GAG CTT CAA GCC AAC AAG GCC ACA CTA GTG TGT    480
Lys Ala Thr Leu Glu Glu Leu Gln Ala Asn Lys Ala Thr Leu Val Cys
                130                 135                 140

CTG ATC AGT GAC TTC TAC CCG GGA GCT GTG ACA TTG GCT TGG AAG GCA    528
Leu Ile Ser Asp Phe Tyr Pro Gly Ala Val Thr Leu Ala Trp Lys Ala
            145                 150                 155

GAT GGC AGG CCC GTC AAG GCG GGA GTG GAG ACC AAC AAA CCC TCC AAA    576
Asp Gly Arg Pro Val Lys Ala Gly Val Glu Thr Asn Lys Pro Ser Lys
        160                 165                 170

CAG AGC AAC AAC AAG TAC GCG GCC AGC AGC TAC CTG AGC CTG ACG CCC    624
Gln Ser Asn Asn Lys Tyr Ala Ala Ser Ser Tyr Leu Ser Leu Thr Pro
        175                 180                 185

GAG CAG TGG AAG TCC CAC AGA AGC TAC AGC TGC CAG GTC ACG CAT GAA    672
Glu Gln Trp Lys Ser His Arg Ser Tyr Ser Cys Gln Val Thr His Glu
190                 195                 200                 205

GGG AGC ACT GCA GAG AAG ACG GTG GCC CCT GCA GAA TGT TCA    TA      716
Gly Ser Thr Ala Glu Lys Thr Val Ala Pro Ala Glu Cys Ser
                210                 215
```

(2) INFORMATIONS POUR LA SEQ ID NO: 6:

    (i) CARACTERISTIQUES DE LA SEQUENCE:

        (A) LONGUEUR: 238 acides aminés
        (B) TYPE: acide aminé

EP 0 778 891 B1

(D) CONFIGURATION: linéaire

(ii) TYPE DE MOLECULE: protéine

(xi) DESCRIPTION DE LA SEQUENCE: SEQ ID NO: 6:

```
Met Gly Trp Ser Cys Ile Ile Leu Phe Leu Val Ala Thr Ala Thr Gly
-19            -15             -10                       -5

Val His Ser Asp Ile Glu Leu Thr Gln Asp Pro Ala Val Ser Val Ala
             1            5              10

Leu Gly Gln Thr Val Arg Ile Thr Cys Gln Gly Asp Ser Leu Arg Thr
        15            20             25

Tyr Tyr Ala Ser Trp Tyr Gln Gln Lys Pro Gly Gln Ala Pro Val Leu
    30            35            40                   45

Val Ile Tyr Gly Lys Asn Asn Arg Pro Ser Gly Ile Pro Asp Arg Phe
            50            55            60

Ser Gly Ser Ser Ser Gly Asn Thr Ala Ser Leu Thr Ile Thr Gly Ala
            65            70            75

Gln Ala Glu Asp Glu Ala Asp Tyr Phe Cys Asn Ser Gly Gly Lys Val
        80            85            90

Phe Gly Gly Gly Thr Lys Leu Thr Val Leu Gly Gln Pro Lys Ala Ala
    95            100           105

Pro Ser Val Thr Leu Phe Pro Pro Ser Leu Glu Glu Leu Gln Ala Asn
110           115           120           125

Lys Ala Thr Leu Glu Glu Leu Gln Ala Asn Lys Ala Thr Leu Val Cys
            130           135           140

Leu Ile Ser Asp Phe Tyr Pro Gly Ala Val Thr Leu Ala Trp Lys Ala
            145           150           155

Asp Gly Arg Pro Val Lys Ala Gly Val Glu Thr Asn Lys Pro Ser Lys
        160           165           170

Gln Ser Asn Asn Lys Tyr Ala Ala Ser Ser Tyr Leu Ser Leu Thr Pro
        175           180           185

Glu Gln Trp Lys Ser His Arg Ser Tyr Ser Cys Gln Val Thr His Glu
190           195           200           205

Gly Ser Thr Ala Glu Lys Thr Val Ala Pro Ala Glu Cys Ser
            210           215
```

(2) INFORMATIONS POUR LA SEQ ID NO: 7:

(i) CARACTERISTIQUES DE LA SEQUENCE:

(A) LONGUEUR: 1418 paires de bases
(B) TYPE: nucléotide
(C) NOMBRE DE BRINS: simple
(D) CONFIGURATION: linéaire

(ix) CARACTERISTIQUE:

(A) NOM/CLE: CDS

21

(B) EMPLACEMENT:1..1418

(ix) CARACTERISTIQUE:

    (A) NOM/CLE: sig_peptide
    (B) EMPLACEMENT:1..57

(ix) CARACTERISTIQUE:

    (A) NOM/CLE: mat_peptide
    (B) EMPLACEMENT:58..1418
    (D) AUTRES INFORMATIONS:/product= "Immunoglobulin, Heavy Chain"

(xi) DESCRIPTION DE LA SEQUENCE: SEQ ID NO: 7:

```
ATG GGA TGG AGC TGT ATC ATC CTC TTC TTG GTA GCA ACA GCT ACA GGT        48
Met Gly Trp Ser Cys Ile Ile Leu Phe Leu Val Ala Thr Ala Thr Gly
-19           -15             -10                 -5

GTC CAC TCC CAG GTC CAA CTG CAG CAG TGG GGC GCA GGA CTG TTG AAG        96
Val His Ser Gln Val Gln Leu Gln Gln Trp Gly Ala Gly Leu Leu Lys
              1               5                  10

CCT TCG GAG ACC CTG TCC CTC ACC TGC ACT GTC TAT GGT GGG TCC TTC       144
Pro Ser Glu Thr Leu Ser Leu Thr Cys Thr Val Tyr Gly Gly Ser Phe
        15                  20                  25

AGT GGT TAC TAC TGG AGC TGG ATC CGC CAG CCC CCA GGG AAG GGG CTG       192
Ser Gly Tyr Tyr Trp Ser Trp Ile Arg Gln Pro Pro Gly Lys Gly Leu
 30                 35                  40                  45

GAG TGG ATT GGG GAA ATC AAT CAT AGT GGA AGC ACC AAC TAC AAC CCG       240
Glu Trp Ile Gly Glu Ile Asn His Ser Gly Ser Thr Asn Tyr Asn Pro
                50                  55                  60
```

```
TCC CTC AAG AGT CGA GTC ACC ATA TCA GTA GAC ACG TCC AAG AAC CAG       288
Ser Leu Lys Ser Arg Val Thr Ile Ser Val Asp Thr Ser Lys Asn Gln
            65              70              75

TTC TCC CTG AAA CTG AAC TCT GTG ACC GCC GCG GAC ACG GCT GTG TAT       336
Phe Ser Leu Lys Leu Asn Ser Val Thr Ala Ala Asp Thr Ala Val Tyr
        80              85              90

TAC TGT GCG AGG GCC CCA GAG TAT AAA TGG AAG TAT CAT GGG GAC TGG       384
Tyr Cys Ala Arg Ala Pro Glu Tyr Lys Trp Lys Tyr His Gly Asp Trp
    95              100             105

TTC GAC CCC TGG GGC CAA GGT ACC ACT GTC ACC GTC TCC TCA GCC TCC       432
Phe Asp Pro Trp Gly Gln Gly Thr Thr Val Thr Val Ser Ser Ala Ser
110             115             120             125

ACC AAG GGC CCA TCG GTC TTC CCC CTG GCA CCC TCC TCC AAG AGC ACC       480
Thr Lys Gly Pro Ser Val Phe Pro Leu Ala Pro Ser Ser Lys Ser Thr
            130             135             140

TCT GGG GGC ACA GCG GCC CTG GGC TGC CTG GTC AAG GAC TAC TTC CCC       528
Ser Gly Gly Thr Ala Ala Leu Gly Cys Leu Val Lys Asp Tyr Phe Pro
        145             150             155

GAA CCG GTG ACG GTG TCG TGG AAC TCA GGC GCC CTG ACC AGC GGC GTG       576
Glu Pro Val Thr Val Ser Trp Asn Ser Gly Ala Leu Thr Ser Gly Val
        160             165             170

CAC ACC TTC CCG GCT GTC CTA CAG TCC TCA GGA CTC TAC TCC CTC AGC       624
His Thr Phe Pro Ala Val Leu Gln Ser Ser Gly Leu Tyr Ser Leu Ser
    175             180             185

AGC GTG GTG ACC GTG CCC TCC AGC AGC TTG GGC ACC CAG ACC TAC ATC       672
Ser Val Val Thr Val Pro Ser Ser Ser Leu Gly Thr Gln Thr Tyr Ile
190             195             200             205

TGC AAC GTG AAT CAC AAG CCC AGC AAC ACC AAG GTG GAC AAG AAA GCA       720
Cys Asn Val Asn His Lys Pro Ser Asn Thr Lys Val Asp Lys Lys Ala
                210             215             220

GAG CCC AAA TCT TGT GAC AAA ACT CAC ACA TGC CCA CCG TGC CCA GCA       768
Glu Pro Lys Ser Cys Asp Lys Thr His Thr Cys Pro Pro Cys Pro Ala
            225             230             235

CCT GAA CTC CTG GGG GGA CCG TCA GTC TTC CTC TTC CCC CCA AAA CCC       816
Pro Glu Leu Leu Gly Gly Pro Ser Val Phe Leu Phe Pro Pro Lys Pro
        240             245             250

AAG GAC ACC CTC ATG ATC TCC CGG ACC CCT GAG GTC ACA TGC GTG GTG       864
Lys Asp Thr Leu Met Ile Ser Arg Thr Pro Glu Val Thr Cys Val Val
        255             260             265

GTG GAC GTG AGC CAC GAA GAC CCT GAG GTC AAG TTC AAC TGG TAC GTG       912
Val Asp Val Ser His Glu Asp Pro Glu Val Lys Phe Asn Trp Tyr Val
270             275             280             285

GAC GGC GTG GAG GTG CAT AAT GCC AAG ACA AAG CCG CGG GAG GAG CAG       960
Asp Gly Val Glu Val His Asn Ala Lys Thr Lys Pro Arg Glu Glu Gln
            290             295             300

TAC AAC AGC ACG TAC CGG GTG GTC AGC GTC CTC AAA GTC CTG CAC CAG       1008
Tyr Asn Ser Thr Tyr Arg Val Val Ser Val Leu Lys Val Leu His Gln
            305             310             315
```

```
GAC TGG CTG AAT GGC AAG GAG TAC AAG TGC AAG GTC TCC AAC AAA GCC      1056
Asp Trp Leu Asn Gly Lys Glu Tyr Lys Cys Lys Val Ser Asn Lys Ala
        320             325             330

CTC CCA GCC CCC ATC GAG AAA ACC ATC TCC AAA GCC AAA GGG CAG CCC      1104
Leu Pro Ala Pro Ile Glu Lys Thr Ile Ser Lys Ala Lys Gly Gln Pro
        335             340             345

CGA GAA CCA CAG GTG TAC ACC CTG CCC CCA TCC CGG GAT GAG CTG ACC      1152
Arg Glu Pro Gln Val Tyr Thr Leu Pro Pro Ser Arg Asp Glu Leu Thr
350             355             360             365

AAG AAC CAG GTC AGC CTG ACC TGC CTG GTC AAA GGC TTC TAT CCC AGC      1200
Lys Asn Gln Val Ser Leu Thr Cys Leu Val Lys Gly Phe Tyr Pro Ser
        370             375             380

GAC ATC GCC GTG GAG TGG GAG AGC AAT GGG CAG CCG GAG AAC AAC TAC      1248
Asp Ile Ala Val Glu Trp Glu Ser Asn Gly Gln Pro Glu Asn Asn Tyr
        385             390             395

AAG ACC ACG CCT CCC GTG CTG GAC TCC GAC GGC TCC TTC TTC CTC TAC      1296
Lys Thr Thr Pro Pro Val Leu Asp Ser Asp Gly Ser Phe Phe Leu Tyr
        400             405             410

AGC AAG CTC ACC GTG GAC AAG AGC AGG TGG CAG CAG GGG AAC GTC TTC      1344
Ser Lys Leu Thr Val Asp Lys Ser Arg Trp Gln Gln Gly Asn Val Phe
        415             420             425

TCA TGC TCC GTG ATG CAT GAG GCT CTG CAC AAC CAC TAC ACG CAG AAG      1392
Ser Cys Ser Val Met His Glu Ala Leu His Asn His Tyr Thr Gln Lys
430             435             440             445

AGC CTC TCC CTG TCT CCG GGT AAA  TG                                  1418
Ser Leu Ser Leu Ser Pro Gly Lys
        450
```

(2) INFORMATIONS POUR LA SEQ ID NO: 8:

    (i) CARACTERISTIQUES DE LA SEQUENCE:

        (A) LONGUEUR: 472 acides aminés
        (B) TYPE: acide aminé
        (D) CONFIGURATION: linéaire

    (ii) TYPE DE MOLECULE: protéine

    (xi) DESCRIPTION DE LA SEQUENCE: SEQ ID NO: 8:

```
Met Gly Trp Ser Cys Ile Ile Leu Phe Leu Val Ala Thr Ala Thr Gly
-19         -15             -10             -5

Val His Ser Gln Val Gln Leu Gln Gln Trp Gly Ala Gly Leu Leu Lys
            1           5               10

Pro Ser Glu Thr Leu Ser Leu Thr Cys Thr Val Tyr Gly Gly Ser Phe
        15          20              25

Ser Gly Tyr Tyr Trp Ser Trp Ile Arg Gln Pro Pro Gly Lys Gly Leu
30              35              40              45

Glu Trp Ile Gly Glu Ile Asn His Ser Gly Ser Thr Asn Tyr Asn Pro
                50              55              60

Ser Leu Lys Ser Arg Val Thr Ile Ser Val Asp Thr Ser Lys Asn Gln
            65              70              75
```

```
Phe Ser Leu Lys Leu Asn Ser Val Thr Ala Ala Asp Thr Ala Val Tyr
        80                  85                  90

Tyr Cys Ala Arg Ala Pro Glu Tyr Lys Trp Lys Tyr His Gly Asp Trp
        95                 100                 105

Phe Asp Pro Trp Gly Gln Gly Thr Thr Val Thr Val Ser Ser Ala Ser
110                 115                 120                 125

Thr Lys Gly Pro Ser Val Phe Pro Leu Ala Pro Ser Ser Lys Ser Thr
                130                 135                 140

Ser Gly Gly Thr Ala Ala Leu Gly Cys Leu Val Lys Asp Tyr Phe Pro
            145                 150                 155

Glu Pro Val Thr Val Ser Trp Asn Ser Gly Ala Leu Thr Ser Gly Val
        160                 165                 170

His Thr Phe Pro Ala Val Leu Gln Ser Ser Gly Leu Tyr Ser Leu Ser
    175                 180                 185

Ser Val Val Thr Val Pro Ser Ser Ser Leu Gly Thr Gln Thr Tyr Ile
190                 195                 200                 205

Cys Asn Val Asn His Lys Pro Ser Asn Thr Lys Val Asp Lys Lys Ala
            210                 215                 220

Glu Pro Lys Ser Cys Asp Lys Thr His Thr Cys Pro Pro Cys Pro Ala
        225                 230                 235

Pro Glu Leu Leu Gly Gly Pro Ser Val Phe Leu Phe Pro Pro Lys Pro
        240                 245                 250

Lys Asp Thr Leu Met Ile Ser Arg Thr Pro Glu Val Thr Cys Val Val
        255                 260                 265

Val Asp Val Ser His Glu Asp Pro Glu Val Lys Phe Asn Trp Tyr Val
270                 275                 280                 285

Asp Gly Val Glu Val His Asn Ala Lys Thr Lys Pro Arg Glu Glu Gln
            290                 295                 300

Tyr Asn Ser Thr Tyr Arg Val Val Ser Val Leu Lys Val Leu His Gln
            305                 310                 315

Asp Trp Leu Asn Gly Lys Glu Tyr Lys Cys Lys Val Ser Asn Lys Ala
        320                 325                 330

Leu Pro Ala Pro Ile Glu Lys Thr Ile Ser Lys Ala Lys Gly Gln Pro
        335                 340                 345

Arg Glu Pro Gln Val Tyr Thr Leu Pro Pro Ser Arg Asp Glu Leu Thr
350                 355                 360                 365

Lys Asn Gln Val Ser Leu Thr Cys Leu Val Lys Gly Phe Tyr Pro Ser
                370                 375                 380

Asp Ile Ala Val Glu Trp Glu Ser Asn Gly Gln Pro Glu Asn Asn Tyr
            385                 390                 395

Lys Thr Thr Pro Pro Val Leu Asp Ser Asp Gly Ser Phe Phe Leu Tyr
        400                 405                 410

Ser Lys Leu Thr Val Asp Lys Ser Arg Trp Gln Gln Gly Asn Val Phe
    415                 420                 425
```

25

```
Ser Cys Ser Val Met His Glu Ala Leu His Asn His Tyr Thr Gln Lys
430                 435                 440                 445

Ser Leu Ser Leu Ser Pro Gly Lys
                450
```

(2) INFORMATIONS POUR LA SEQ ID NO: 9:

    (i) CARACTERISTIQUES DE LA SEQUENCE:

        (A) LONGUEUR: 24 paires de bases
        (B) TYPE: nucléotide
        (C) NOMBRE DE BRINS: simple
        (D) CONFIGURATION: linéaire

    (xi) DESCRIPTION DE LA SEQUENCE: SEQ ID NO: 9:

```
CCTCAGTGAA GGTCTCCTGC AAGG                                    24
```

(2) INFORMATIONS POUR LA SEQ ID NO: 10:

    (i) CARACTERISTIQUES DE LA SEQUENCE:

        (A) LONGUEUR: 24 paires de bases
        (B) TYPE: nucléotide
        (C) NOMBRE DE BRINS: simple
        (D) CONFIGURATION: linéaire

    (xi) DESCRIPTION DE LA SEQUENCE: SEQ ID NO: 10:

```
TCCTGCGCTG GTGAAAGCCA CACA                                    24
```

(2) INFORMATIONS POUR LA SEQ ID NO: 11:

    (i) CARACTERISTIQUES DE LA SEQUENCE:

        (A) LONGUEUR: 23 paires de bases
        (B) TYPE: nucléotide
        (C) NOMBRE DE BRINS: simple
        (D) CONFIGURATION: linéaire

    (xi) DESCRIPTION DE LA SEQUENCE: SEQ ID NO: 11:

```
GGTCCCTGAG ACTCTCCTGT GCA                                     23
```

(2) INFORMATIONS POUR LA SEQ ID NO: 12:

    (i) CARACTERISTIQUES DE LA SEQUENCE:

        (A) LONGUEUR: 25 paires de bases
        (B) TYPE: nucléotide
        (C) NOMBRE DE BRINS: simple
        (D) CONFIGURATION: linéaire

(xi) DESCRIPTION DE LA SEQUENCE: SEQ ID NO: 12:

TCGGAGACCC TGTCCCTCAC CTGCA                                                        25

(2) INFORMATIONS POUR LA SEQ ID NO: 13:

    (i) CARACTERISTIQUES DE LA SEQUENCE:

        (A) LONGUEUR: 24 paires de bases
        (B) TYPE: nucléotide
        (C) NOMBRE DE BRINS: simple
        (D) CONFIGURATION: linéaire

    (xi) DESCRIPTION DE LA SEQUENCE: SEQ ID NO: 13:

CGCTGTCTCT GGTTACTCCA TCAG                                                         24

(2) INFORMATIONS POUR LA SEQ ID NO: 14:

    (i) CARACTERISTIQUES DE LA SEQUENCE:

        (A) LONGUEUR: 24 paires de bases
        (B) TYPE: nucléotide
        (C) NOMBRE DE BRINS: simple
        (D) CONFIGURATION: linéaire

    (xi) DESCRIPTION DE LA SEQUENCE: SEQ ID NO: 14:

GAAAAAGCCC GGGGAGTCTC TGAA                                                         24

(2) INFORMATIONS POUR LA SEQ ID NO: 15:

    (i) CARACTERISTIQUES DE LA SEQUENCE:

        (A) LONGUEUR: 24 paires de bases
        (B) TYPE: nucléotide
        (C) NOMBRE DE BRINS: simple
        (D) CONFIGURATION: linéaire

    (xi) DESCRIPTION DE LA SEQUENCE: SEQ ID NO: 15:

CCTGTGCCAT CTCCGGGGAC AGTG                                                         24

(2) INFORMATIONS POUR LA SEQ ID NO: 16:

    (i) CARACTERISTIQUES DE LA SEQUENCE:

        (A) LONGUEUR: 18 paires de bases
        (B) TYPE: nucléotide
        (C) NOMBRE DE BRINS: simple
        (D) CONFIGURATION: linéaire

    (xi) DESCRIPTION DE LA SEQUENCE: SEQ ID NO: 16:

```
CAGTCTGTGC TGACTCAG                                              18
```

(2) INFORMATIONS POUR LA SEQ ID NO: 17:

    (i) CARACTERISTIQUES DE LA SEQUENCE:

        (A) LONGUEUR: 20 paires de bases
        (B) TYPE: nucléotide
        (C) NOMBRE DE BRINS: simple
        (D) CONFIGURATION: linéaire

    (xi) DESCRIPTION DE LA SEQUENCE: SEQ ID NO: 17:

```
CACACYAGTG TRGCCTGGTT                                            20
```

.

(2) INFORMATIONS POUR LA SEQ ID NO : 18:

    (1) CARACTERISTIQUES DE LA SEQUENCE:

        (A) LONGUEUR: 30 paires de bases
        (B) TYPE: nucléotide
        (C) NOMBRE DE BRINS: simple
        (D) CONFIGURATION: linéaire

    (xi) DESCRIPTION DE LA SEQUENCE: SEQ ID NO: 18:

```
CCTGTCAGAT CTATGAACAT TCTGTAGGGG                                 30
```

(2) INFORMATIONS POUR LA SEQ ID NO: 19:

    (i) CARACTERISTIQUES DE LA SEQUENCE:

        (A) LONGUEUR: 21 paires de bases
        (B) TYPE: nucléotide
        (C) NOMBRE DE BRINS: simple
        (D) CONFIGURATION: linéaire

    (xi) DESCRIPTION DE LA SEQUENCE: SEQ ID NO: 19:

```
CCGCCCTCCC TCGAGCTTCA A                                          21
```

(2) INFORMATIONS POUR LA SEQ ID NO: 20:

    (i) CARACTERISTIQUES DE LA SEQUENCE:

        (A) LONGUEUR: 18 paires de bases
        (B) TYPE: nucléotide
        (C) NOMBRE DE BRINS: simple
        (D) CONFIGURATION: linéaire

    (xi) DESCRIPTION DE LA SEQUENCE: SEQ ID NO: 20:

CASTCTGAGC TCACKCAG                                                          18

(2) INFORMATIONS POUR LA SEQ ID NO: 21:

    (i) CARACTERISTIQUES DE LA SEQUENCE:

      (A) LONGUEUR: 24 paires de bases
      (B) TYPE: nucléotide
      (C) NOMBRE DE BRINS: simple
      (D) CONFIGURATION: linéaire

    (xi) DESCRIPTION DE LA SEQUENCE: SEQ ID NO: 21:

TTGAAGCTCG AGGGAGGGCG GGAA                                                   24

(2) INFORMATIONS POUR LA SEQ ID NO: 22:

    (i) CARACTERISTIQUES DE LA SEQUENCE:

      (A) LONGUEUR: 24 paires de bases
      (B) TYPE: nucléotide
      (C) NOMBRE DE BRINS: simple
      (D) CONFIGURATION: linéaire

    (xi) DESCRIPTION DE LA SEQUENCE: SEQ ID NO: 22:

CAAGGTACCA CGGTCACCGT CTCC                                                   24

(2) INFORMATIONS POUR LA SEQ ID NO: 23:

    (i) CARACTERISTIQUES DE LA SEQUENCE:

      (A) LONGUEUR: 29 paires de bases
      (B) TYPE: nucléotide
      (C) NOMBRE DE BRINS: simple
      (D) CONFIGURATION: linéaire

    (xi) DESCRIPTION DE LA SEQUENCE: SEQ ID NO: 23:

GAAGATCTCA TTTACCCGGA GACAGGGAG                                              29

(2) INFORMATIONS POUR LA SEQ ID NO: 24:

    (i) CARACTERISTIQUES DE LA SEQUENCE:

      (A) LONGUEUR: 57 paires de bases
      (B) TYPE: nucléotide
      (C) NOMBRE DE BRINS: simple
      (D) CONFIGURATION: linéaire

    (xi) DESCRIPTION DE LA SEQUENCE: SEQ ID NO: 24:

```
GTCCAACTGC AGCAGTGGGG CGCAGGACTG TTGAAGCCTT CGGAGACCCT GTCCCTC          57
```

(2) INFORMATIONS POUR LA SEQ ID NO: 25:

    (i) CARACTERISTIQUES DE LA SEQUENCE:

       (A) LONGUEUR: 29 paires de bases
       (B) TYPE: nucléotide
       (C) NOMBRE DE BRINS: simple
       (D) CONFIGURATION: linéaire

    (xi) DESCRIPTION DE LA SEQUENCE: SEQ ID NO: 25:

```
TGAGGAGACG GTGACCGTGG TACCTTGGC          29
```